(19) Europäisches Patentamt / European Patent Office / Office européen des brevets

(11) **EP 3 483 744 B1**

(12) **EUROPEAN PATENT SPECIFICATION**

(45) Date of publication and mention of the grant of the patent:
**23.03.2022 Bulletin 2022/12**

(21) Application number: **17824149.3**

(22) Date of filing: **30.06.2017**

(51) International Patent Classification (IPC):
$G06F\ 17/18^{(2006.01)}$    $G06F\ 21/62^{(2013.01)}$
$H04L\ 9/00^{(2022.01)}$    $G16B\ 40/00^{(2019.01)}$

(52) Cooperative Patent Classification (CPC):
**G06F 17/18; G06F 21/6245; G16B 40/00; G16B 50/00**

(86) International application number:
**PCT/JP2017/024128**

(87) International publication number:
**WO 2018/008544 (11.01.2018 Gazette 2018/02)**

(54) **FISHER'S EXACT TEST CALCULATION APPARATUS, METHOD, AND PROGRAM**

VORRICHTUNG, VERFAHREN UND PROGRAMM ZUR BERECHNUNG VOM EXAKTEN TEST NACH FISHER

APPAREIL, PROCÉDÉ ET PROGRAMME DE CALCUL DE TEST EXACT DE FISHER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.07.2016 JP 2016134087**

(43) Date of publication of application:
**15.05.2019 Bulletin 2019/20**

(73) Proprietors:
• **Nippon Telegraph and Telephone Corporation**
**Tokyo 100-8116 (JP)**
• **Tohoku University**
**Sendai-shi, Miyagi 980-8577 (JP)**

(72) Inventors:
• **HASEGAWA, Satoshi**
**Musashino-shi,**
**Tokyo 180-8585 (JP)**
• **HAMADA, Koki**
**Musashino-shi,**
**Tokyo 180-8585 (JP)**
• **CHIDA, Koji**
**Musashino-shi,**
**Tokyo 180-8585 (JP)**
• **NAGASAKI, Masao**
**Sendai-shi,**
**Miyagi 980-8577 (JP)**

• **MISAWA, Kazuharu**
**Sendai-shi,**
**Miyagi 980-8577 (JP)**

(74) Representative: **MERH-IP Matias Erny Reichl Hoffmann**
**Patentanwälte PartG mbB**
**Paul-Heyse-Strasse 29**
**80336 München (DE)**

(56) References cited:
**WO-A1-2007/126088    JP-A- 2009 086 739**

• **DAN BOGDANOV ET AL: "Rmind: a tool for cryptographically secure statistical analysis", IACR, INTERNATIONAL ASSOCIATION FOR CRYPTOLOGIC RESEARCH, vol. 20140630:165415, 30 June 2014 (2014-06-30), pages 1-35, XP061016368,**
• **LIINA KAMM ET AL: "A new way to protect privacy in large-scale genome-wide association studies", BIOINFORMATICS., vol. 29, no. 7, 14 February 2013 (2013-02-14), pages 886-893, XP055654646, GB ISSN: 1367-4803, DOI: 10.1093/bioinformatics/btt066**

- HAMADA ET AL: "Privacy-Preserving Fisher's Exact Test for Genome-Wide Association Study", INTERNATIONAL WORKSHOP ON GENOME PRIVACY AND SECURITY (GENOPRI '17) , 15-10-2017; ORLANDO, FL, USA, GLOBAL ALLIANCE FOR GENOMICS AND HEALTH, US , 15 October 2017 (2017-10-15), pages 1-20, XP009516359, Retrieved from the Internet: URL:http://2017.genopri.org/uploads/3/9/9/9/39999711/genopri17_paper_5.pdf
- YIHUA ZHANG ET AL: "Secure distributed genome analysis for GWAS and sequence comparison computation", BMC MEDICAL INFORMATICS AND DECISION MAKING, vol. 15, no. S5, December 2015 (2015-12), XP055748476, DOI: 10.1186/1472-6947-15-S5-S4
- FELIPE LLINARES L\'OPEZ ET AL: "Fast and Memory-Efficient Significant Pattern Mining via Permutation Testing", ARXIV.ORG, CORNELL UNIVERSITY LIBRARY, 201 OLIN LIBRARY CORNELL UNIVERSITY ITHACA, NY 14853, 15 February 2015 (2015-02-15), XP080677993,
- HAMADA KOKI ET AL: "Privacy Preserving Fisher's Exact Test (2) - For Large Samples", RESEARCH REPORT OF INFORMATION PROCESSING SOCIETY OF JAPAN, INFORMATION PROCESSING SOCIETY OF JAPAN, JP, vol. 2016 -CSEC-74, no. 38, 7 July 2016 (2016-07-07), pages 1-6, XP009513204, ISSN: 0919-6072
- Ali Shahbazi ET AL: "Private computation with genomic data for genome-wide association and linkage studies", GenoPri Workshop 2016, 12 November 2016, Chicago, US, 12 November 2016 (2016-11-12), XP055748519, Retrieved from the Internet: URL:https://www.acsu.buffalo.edu/~mblanton/publications/genopri16.pdf [retrieved on 2020-11-09]
- DAMGÅRD IVAN ET AL: "Unconditionally Secure Constant-Rounds Multi-party Computation for Equality, Comparison, Bits and Exponentiation", 4 March 2006 (2006-03-04), BIG DATA ANALYTICS IN THE SOCIAL AND UBIQUITOUS CONTEXT : 5TH INTERNATIONAL WORKSHOP ON MODELING SOCIAL MEDIA, MSM 2014, 5TH INTERNATIONAL WORKSHOP ON MINING UBIQUITOUS AND SOCIAL ENVIRONMENTS, MUSE 2014 AND FIRST INTERNATIONAL WORKSHOP ON MACHINE LE, XP047422636, ISBN: 978-3-642-17318-9
- TAKASHI NISHIDE ET AL: "Multiparty Computation for Interval, Equality, and Comparison Without Bit-Decomposition Protocol", 16 April 2007 (2007-04-16), PUBLIC KEY CRYPTOGRAPHY - PKC 2007; [LECTURE NOTES IN COMPUTER SCIENCE;;LNCS], SPRINGER BERLIN HEIDELBERG, BERLIN, HEIDELBERG, PAGE(S) 343 - 360, XP047029618, ISBN: 978-3-540-71676-1
- Konrad Karczewski: "How to do a GWAS", GENE 210: Genomics and Personalized Medicine, 1 January 2015 (2015-01-01), XP055749998, Retrieved from the Internet: URL:https://stanford.edu/class/gene210/files/gwas-howto.pdf [retrieved on 2020-11-12]
- NARUMANCHI HARIKA ET AL: "Search over Compute: Solving Multiplication-Intensive Computational Problems over FHE Data", 2018 IEEE INTERNATIONAL CONFERENCE ON SMART CLOUD (SMARTCLOUD), IEEE, 21 September 2018 (2018-09-21), pages 40-45, XP033431316, DOI: 10.1109/SMARTCLOUD.2018.00015 [retrieved on 2018-10-26]
- CRAIG GENTRY: "Fully homomorphic encryption using ideal lattices", PROCEEDINGS OF THE 2009 ACM INTERNATIONAL SYMPOSIUM ON THEORY OF COMPUTING : BETHESDA, MARYLAND, USA, MAY 31 - JUNE 2, 2009, ASSOCIATION FOR COMPUTING MACHINERY, 2 PENN PLAZA, SUITE 701 NEW YORK NY 10121-0701 USA, 31 May 2009 (2009-05-31), pages 169-178, XP058164708, DOI: 10.1145/1536414.1536440 ISBN: 978-1-60558-506-2

**Description**

[TECHNICAL FIELD]

The present invention relates to techniques for efficiently and securely calculating Fisher's exact test.

[BACKGROUND ART]

Fisher's exact test is widely known as one of statistical test methods. An application of Fisher's exact test is genome-wide association study (GWAS) (see Non-patent Literature 1, for instance). Brief description of Fisher's exact test is given below.

Table 1

|       | X   | Y   | Total          |
|-------|-----|-----|----------------|
| A     | a   | b   | a+b            |
| G     | c   | d   | c+d            |
| Total | a+c | b+d | a+b+c+d (=n)   |

This table is an example of a 2x2 contingency table (summary table) that classifies n subjects according to character (X or Y) and a particular allele (A or G) and counts the results, where a, b, c, and d represent frequencies (non-negative integers). In Fisher's exact test, when the following is assumed for a non-negative integer i,

$$p_i = \frac{(a + b)!\,(c + d)!\,(a + c)!\,(b + d)!}{n!\,i!\,(a + b - i)!\,(a + c - i)!\,(d - a + i)!}$$

it is determined whether there is a statistically significant association between the character and a particular allele based on the magnitude relationship between:

$$p = p_a + \sum_{\substack{\max(0,a-d)\le i\le \min(a+b,a+c) \\ p_i < p_a}} p_i$$

and a threshold T of a predetermined value. In genome-wide association study, a contingency table like the above one can be created for each single nucleotide polymorphism (SNP) and Fisher's exact test can be performed on each one of the contingency tables. Genome-wide association study involves an enormous number of SNPs on the order of several millions to tens of millions. Thus, in genome-wide association study, there can be a situation where a large quantity of Fisher's exact test is performed.

Meanwhile, in view of the sensitivity or confidentiality of genome information, some prior studies are intended to perform genome-wide association study while concealing genome information via encryption techniques (see Non-patent Literature 2, for instance). Non-patent Literature 2 proposes a method of performing a chi-square test while concealing genome information.

Non-patent Literature 1 referenced above specifically relates to techniques that use genome-wide association studies for discovering genetic factors that influence diseases. In these approaches, hundreds of thousands or millions of genetic loci are probed in a set of cases and controls of a disease. For each genetic locus, the association between genotype and phenotype is measured by a chi-squared (or Fisher's exact) test. A correction factor is then applied to the p-value of each association to discover "genome-wide" significant variants.

Non-patent Literature 3 relates to secure multi-party computation platforms and privacy-preserving statistical analysis using secure multi-party computation, and describes the most used statistical analysis functions in the privacy-preserving setting including simple statistics, t-test, $\chi 2$ test, Wilcoxon tests and linear regression.

[PRIOR ART LITERATURE]

[NON-PATENT LITERATURE]

Non-patent Literature 1: Konrad Karczewski, "How to do a GWAS", GENE 210: Genomics and Personalized Medicine, 2015.
Non-patent Literature 2: Yihua Zhang, Marina Blanton, and Ghada Almashaqbeh, "Secure distributed genome analysis for GWAS and sequence comparison computation", BMC medical informatics and decision making, Vol. 15, No. Suppl 5, p. S4, 2015.
Non-patent Literature 3: Dan Bogdanov, Liina Kamm, Sven Laur, Ville Sokk, "Rmind: a tool for cryptographically secure statistical analysis", IACR, INTERNATIONAL ASSOCIATION FOR CRYPTOLOGIC RESEARCH, 30 June 2014, pp. 1-35.

[SUMMARY OF THE INVENTION]

[PROBLEMS TO BE SOLVED BY THE INVENTION]

Since a single execution of Fisher's exact test requires calculation of a maximum of n/2 types of $p_i$ and Fisher's exact test can be conducted on individual ones of a large quantity of contingency tables in the case of genome-wide association study in particular, it could involve an enormous processing time depending on the computer environment and/or the frequencies in the contingency tables.

An object of the present invention is to provide a Fisher's exact test calculation apparatus, method, and program for performing calculations for multiple executions of Fisher's exact test in a more efficient manner than conventional arts, while keeping the frequencies in the plurality of contingency tables concealed.

[MEANS TO SOLVE THE PROBLEMS]

In view of the above problems, the present invention provides Fisher's exact test calculation apparatus, computer-implemented methods of performing Fisher's exact test for a plurality of 2x2 contingency tables, and corresponding programs, having the features of respective independent claims.

A Fisher's exact test calculation apparatus according to an example that is not encompassed by the claims but useful for understanding the present invention includes a selection unit that selects contingency tables for which a result of Fisher's exact test indicative of being significant will be possibly obtained from among a plurality of contingency tables based on a parameter obtained in calculation in course of determining the result of Fisher's exact test, and a calculation unit that performs calculations for Fisher's exact test for each of the selected contingency tables.

[EFFECTS OF THE INVENTION]

The present invention can perform calculations for multiple executions of Fisher's exact test in a more efficient manner than conventional arts. More specifically, effects such as a reduced usage of calculation resources and/or a shortened processing time are expected to be achieved. The present invention can also perform these calculations while keeping the frequencies in the plurality of contingency tables concealed.

[BRIEF DESCRIPTION OF THE DRAWINGS]

Fig. 1 is a block diagram for describing an example of a Fisher's exact test calculation apparatus.
Fig. 2 is a flow diagram for describing an example of a Fisher's exact test calculation method.

[DETAILED DESCRIPTION OF THE EMBODIMENT]

An example that is not encompassed by the claims but useful for understanding the present invention is described below with reference to the drawings.

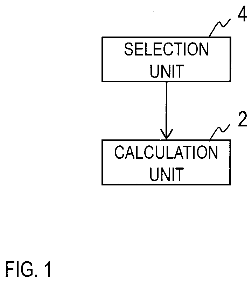

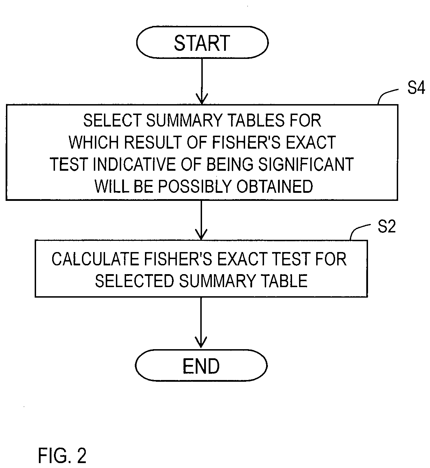

As shown in Fig. 1, a Fisher's exact test calculation apparatus includes a selection unit 4 and a calculation unit 2, for example. A Fisher's exact test calculation method is implemented by these units of the Fisher's exact test calculation apparatus performing the processing at steps S4 and S2 described in Fig. 2 and below.

<Selection unit 4>

The present Fisher's exact test calculation apparatus and method do not perform calculations for Fisher's exact test for each of m contingency tables, where m is a positive integer. Instead, they are given a conditional expression of a sufficient condition under which a result of Fisher's exact test ("TRUE" indicative of having statistically significant association if p is below a threshold T representing a significance level; "FALSE" otherwise) is FALSE, for example. Then, any contingency table that does not satisfy this conditional expression, in other words, any contingency table for which the result of Fisher's exact test will be certainly FALSE, is discarded. For the discarded contingency tables, calculation of the p value is not performed; calculations for Fisher's exact test are performed only for contingency tables that have not been discarded, in other words, contingency tables for which a result of Fisher's exact test indicative of being significant will be possibly obtained.

To this end, the selection unit 4 first selects contingency tables for which a result of Fisher's exact test indicative of being significant will be possibly obtained from multiple contingency tables (m contingency tables) based on a parameter obtained in calculation in the course of determining the result of Fisher's exact test (step S4). Information on the selected contingency tables is output to the calculation unit 2.

An example of a conditional expression for a sufficient condition under which the result of Fisher's exact test will be FALSE is $p_a \geq T$ (or $p_a > T$). Here, $p_a$ represents $p_i$ when i=a, and is defined by the formula below:

$$p_a = \frac{(a+b)!(c+d)!(a+c)!(b+d)!}{n!a!b!c!d!}$$

From the definition of p, $p \geq T$ will always hold when $p_a \geq T$. Accordingly, $p_a \geq T$ can be said to be a conditional expression for a sufficient condition under which the result of Fisher's exact test will be FALSE.

In a case where the conditional expression for the sufficient condition under which the result of Fisher's exact test will be FALSE is $p_a \geq T$, the selection unit 4 calculates $p_a$ based on the frequencies in each contingency table, determines whether $p_a \geq T$, and selects contingency tables for which $p_a \geq T$ does not hold in the determination, in other words, those contingency tables with $p_a < T$.

<Calculation unit 2>

The calculation unit 2 performs calculations for Fisher's exact test for each of the contingency tables selected by the selection unit 4 (step S2). For calculations for Fisher's exact test, any of the existing calculation methods may be employed.

Since a single execution of Fisher's exact test requires calculation of a maximum of n/2 types of $p_i$, the number of calculations is reduced to 2/n at maximum if only the calculation of $p_a$ has to be done. When n=1000, the number of calculations will be reduced to 1/500. However, as Fisher's exact test needs to be performed for contingency tables with $p_a < T$, the lower the ratio of the contingency table with $p_a < T$ is, the more convenient it will be. The table below is the result of an actual experiment which was conducted with contingency tables of genome data (data publicly available without restriction) registered in the NBDC Human Database (Reference Literature 1) for open publication:

Table 2

|  | The number of contingency tables satisfying $p < 5.0 \times 10^{-8}$ | The number of contingency tables satisfying $p_a < 5.0 \times 10^{-8}$ |
|---|---|---|
| Data 1 | 7 | 13 |
| Data 2 | 101 | 133 |
| Data 3 | 33 | 43 |
| Data 4 | 91 | 104 |

The data utilized in the experiment (data 1 to 4) are given in the table below.

Table 3

| Data No. | Disease | Case (# of people) | Control group (# of people) | The number of SNPs (M) | Accession No. |
|---|---|---|---|---|---|
| Data 1 | Cardiac infarction | 1,666 | 3,198 | 455,781 | hum0014.v1.freq.v1 |
| Data 2 | Type 2 diabetes | 9,817 | 6,763 | 552,915 | hum0014.v3.T2DM-1.v1 |
| Data 3 | Type 2 diabetes | 5,645 | 19,420 | 479,088 | hum0014.v3.T2DM-2.v1 |
| Data 4 | Stevens-Johnson syndrome | 117 | 691 | 449,205 | hum0029.v1.freq.v1 |

For data 1 as an example, the ratio of contingency tables with $p_a<T$ is as sufficiently small as $13/455781\approx0.00285\%$, and when assuming that the number of calculations for determining p is n/2 times the number of calculations of $p_a$, the number of calculations for determining p for all SNPs by a common method will be $M\times n/2=455781\times(1666+3198)/2=1,108,459,392$ times the number of calculations of $p_a$. In contrast, when the contingency tables with $p_a<T$ are determined and only p's for those contingency tables are determined according to the present invention, the number of calculations will be $M+L\times n/2=455781+13\times(1666+3198)/2=519,013$ times the number of calculations of $p_a$; the number of calculations is as low as about $519,013/1,108,459,392\approx1/2135.7$, compared to the number of calculations required for determining p's for all SNPs by a common method. Here, M is the number of SNPs and L is the number of contingency tables with $p_a<T$.

Reference Literature 1: NBDC Human Database, the Internet <URL: http://humandbs.biosciencedbc.jp/>

The data used in the experiment were acquired by the Made-to-order Medicine Realization Project (represented by Yusuke Nakamura, director of the RIKEN Center for Genome Medical Sciences), the Made-to-order Medicine Realization Program (represented by Michiaki Kubo, vice director of the RIKEN Center for Integrative Medical Sciences), and the Frontier Medical Science and Technology for Ophthalmology (represented by Mayumi Ueta, an associate professor of Medical Study Department of Kyoto Prefectural University of Medicine) and provided through the "National Bioscience Database Center (NBDC)" website (http://humandbs.biosciencedbc.jp/) of the Japan Science and Technology Agency (JST).

In the present invention, the calculation unit 2 performs calculations for obtaining the result of Fisher's exact test corresponding to the frequencies (a, b, c, d) in the input contingency table subjected to Fisher's exact test while keeping the frequencies (a, b, c, d) concealed via multi-party secure computation. This secure computation can be carried out with the existing secure computation techniques described in Reference Literatures 2 and 3, for example.

Reference Literature 2: Ivan Damgard, Matthias Fitzi, Eike Kiltz, Jesper Buus Nielsen and Tomas Toft, "Unconditionally secure constant-rounds multi-party computation for equality, comparison, bits and exponentiation", In Proc. 3rd Theory of Cryptography Conference, TCC 2006, volume 3876 of Lecture Notes in Computer Science, pages 285-304, Berlin, 2006, Springer-Verlag

Reference Literature 3: Takashi Nishide, Kazuo Ohta, "Multiparty Computation for Interval, Equality, and Comparison Without Bit-Decomposition Protocol", Public Key Cryptography - PKC 2007, 10th International Conference on Practice and Theory in Public-Key Cryptography, 2007, P.343-360

By thus performing calculations for Fisher's exact test only for contingency tables for which a result of Fisher's exact test indicative of being significant will be possibly obtained, in other words, by not performing computation of p for contingency tables for which the result of Fisher's exact test will be obviously FALSE, the amount of calculation for Fisher's exact test on multiple contingency tables can be decreased.

As to the effect of reduction in computation, since $p_a$ is calculated as:

$$p_a = \frac{(a+b)!(c+d)!(a+c)!(b+d)!}{n!\,a!\,b!\,c!\,d!}$$

hence,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j - \sum_{j=1}^{c} \log j - \sum_{j=1}^{d} \log j \quad \textit{... Formula A}$$

thus, by precomputing $\sum_{j=1}^{k} \log j$ for k=0, 1, 2, ..., n and determining $\log p_a$ with the precomputed value, it can be calculated just by additions and subtractions of precomputed values. It may be then determined whether $\log p_a \geq \log T$. Here, $\sum_{j=1}^{0} \log j = 0$ holds.

The selection unit 4 performs the selection of contingency tables described above while keeping the frequencies in multiple contingency tables concealed via multi-party secure computation.

That is, the selection unit 4 performs calculations for determining whether the result of Fisher's exact test satisfies the conditional expression for the sufficient condition under which the result will be FALSE, while concealing the input and output.

Such a calculation is carried out by precomputing $\sum_{j=1}^{k} \log j$ for k=0, 1, 2, ..., n and combining encryption techniques capable of magnitude comparison, determination of equality, and addition/subtraction and multiplication while concealing the input and output. In the following, magnitude comparison with the input and output concealed (hereinafter abbreviated as input/output-concealed magnitude comparison) is described. Assume that two values x and y for magnitude comparison are the input and at least one of x and y is encrypted such that its real numerical value is not known. In the present description, only x is encrypted, which is denoted as E(x). The result of magnitude comparison, which is to be output, is defined as:

$$z = \begin{cases} 1 & \text{if } x \geq y \\ 0 & \text{otherwise} \end{cases}$$

That is to say, the input/output-concealed magnitude comparison means determining cipher text E(z) for the result of magnitude comparison by using E(x),y as the input and without decrypting E(x). When z is the result to be finally obtained, E(z) is appropriately decrypted. Examples of such input/output-concealed magnitude comparison are the methods described in Reference Literature 2 and 3, for example. Similarly, in the case of determination of equality, z will be z=1 if x=y. Examples of this are also the methods of Reference Literature 2 and 3.

A specific example of calculation of $\log p_a$ in Formula A is given. The input is E(a+b), E(c+d), E(a+c), E(b+d), E(n), E(a), E(b), E(c), E(d), and the output is E(z), where z is 1 when $\log p_a \geq \log T$, otherwise 0. This will be described for the first term on the right side of Formula A as an example. First, the precomputed value, $\sum_{j=1}^{k} \log j$ (k=0, 1, ..., n), is used to perform secure computation for determination of equality which returns E(1) if a+b=k and E(0) otherwise. Assume that c=1 if a+b=k and c=0 otherwise. Then, by multiplication secure computation, $E(c\sum_{j=1}^{k} \log j)$ is calculated for each k from E(c) and from $\sum_{j=1}^{k} \log j$.

By finally adding the results while keeping them encrypted, $E(\sum_{j=1}^{a+b} \log j)$ can be obtained. A similar process is then performed for each term on the right side of Formula A and the results are added while being kept encrypted, thus allowing Formula A to be calculated by secure computation.

Assume that the input to the conditional expression for the sufficient condition under which the result of Fisher's exact test will be FALSE is the frequencies, $a_i$, $b_i$, $c_i$, $d_i$, in each contingency table i (i=1, 2, ..., m) and the output is either $TRUE_i$' or $FALSE_i$'. $TRUE_i$' or $FALSE_i$' is denoted as $X_i$'. The input/output in a concealed state is represented by the symbol E(). That is to say, $a_i$ and $TRUE_i$', for example, in a concealed state will be represented as E(ai) and $E(TRUE_i')$, respectively. An operation for returning them from a concealed state to the original state (for example, from $E(a_i)$ to $a_i$) will be referred to as decryption. Then, the result of whether each contingency table satisfies the conditional expression in question, namely $X_i$', can give information on the input, $a_i$, $b_i$, $c_i$, $d_i$.

Accordingly, the selection unit 4 may perform the processes of Examples 1 to 3 described below.

(Example 1)

The selection unit 4 first determines $E(X_i')$ from $E(a_i)$, $E(b_i)$, $E(c_i)$, $E(d_i)$ using input/output-concealed magnitude comparison, and thereafter randomly shuffles the order of m sets, $(E(a_1), E(b_1), E(c_1), E(d_1), E(X_1'))$, $(E(a_2), E(b_2), E(C_2), E(d_2), E(X_2'))$, ..., $(E(a_m), E(b_m), E(c_m), E(d_m), E(X_m'))$, while concealing the shuffled order. The selection unit 4 then decrypts $E(X_i')$ and selects contingency tables corresponding to sets for which the result of decryption has been $TRUE_i$'.

In this case, the calculation unit 2 performs calculations for Fisher's exact test using $E(a_i)$, $E(b_i)$, $E(c_i)$, $E(d_i)$ as the input, in other words, while concealing the input, for the selected contingency tables.

With the scheme of Example 1, selection by the selection unit 4 and calculations for Fisher's exact test by the calculation unit 2 can be performed while concealing the frequencies (a, b, c, d) in contingency tables for which $TRUE_i$'

has been determined.

(Example 2)

In Example 2, the number U of contingency tables to be selected is predetermined.

In a similar manner to Example 1, the selection unit 4 calculates $E(X_i')$ from $E(a_i)$, $E(b_i)$, $E(c_i)$, $E(d_i)$ using input/output-concealed magnitude comparison to determine m sets, $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$. The selection unit 4 then sorts the m sets, $(E(a_1), E(b_i), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$, while concealing $X_i'$, such that $TRUE_i'$ is located at the top or the end. For sorting such that $TRUE_i'$ is located at the top or the end, 1 may be set as a flag indicative of $TRUE_i'$ and 0 may be set as a flag indicative of $FALSE_i'$, for example.

The selection unit 4 then selects U sets from the top or the end of the m sets after being sorted. U is a positive integer.

In this case, the calculation unit 2 performs calculations for Fisher's exact test using $E(a_i)$, $E(b_i)$, $E(c_i)$, $E(d_i)$ as the input, in other words, while concealing the input, for a selected contingency table.

The scheme of Example 2 provides the benefit of enabling further concealment of the number of contingency tables for which $TRUE_i'$ has been determined, in addition to the benefit of the scheme of Example 1.

(Example 3)

In Example 3, the selection unit 4 first calculates $E(X_i')$ from $E(a_i)$, $E(b_i)$, $E(c_i)$, $E(d_i)$ using input/output-concealed magnitude comparison to determine m sets, $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$, in a similar manner to Example 1.

The selection unit 4 then probabilistically replaces $FALSE_i'$ with $TRUE_i'$ while concealing them. An exemplary method for probabilistic replacement with $TRUE_i'$ is to prepare m pieces of data, $E(Y_1')$, $E(Y_2')$, ..., $E(Y_m')$, for which TRUE' or FALSE' is probabilistically concealed in advance, and calculate, for $E(X_i')$, $E(Y_i')$ (i=1, 2, ..., m) and while concealing $X_i'$, $Y_i'$,

$$ Z_i' = \begin{cases} \text{TRUE'} & \text{if } X_i' = \text{TRUE}_i' \text{ OR } Y_i' = \text{TRUE'} \\ \text{FALSE'} & \text{Otherwise} \end{cases} $$

The ratio of TRUE' is appropriately adjusted for $Y_1'$, $Y_2'$, ..., $Y_m'$ so that the number of contingency tables for which $X_i'$ will be actually TRUE' is difficult to infer from the number of contingency tables for which $Z_i'$ is TRUE'.

After the replacement, the selection unit 4 performs a similar process to Example 1.

The scheme of Example 3 provides the benefit of enabling further concealment of the number of contingency tables for which $TRUE_i'$ has been determined, in addition to the benefit of the scheme of Example 1.

Such concealment enables Fisher's exact test to be executed while concealing genome information and various kinds of associated data, for example. This allows, for example, multiple research institutions to obtain the result of executing Fisher's exact test on combined data while concealing the genome data possessed by the individual institutions and without revealing it to one another, which potentially leads to provision of execution environments for genome analysis of an extremely high security level and hence further development of medicine.

[Program and recording medium]

The processes described in connection with the Fisher's exact test calculation apparatus and method may be executed not only in a chronological order in accordance with the order of their description but in a parallel manner or separately depending on the processing ability of the apparatus executing the processes or any necessity.

Also, when the processes of the Fisher's exact test calculation apparatus are to be implemented by a computer, the processing specifics of the functions to be provided by the Fisher's exact test calculation apparatus are described by a program. By the program then being executed by the computer, the processes are embodied on the computer.

The program describing the processing specifics may be recorded on a computer-readable recording medium. The computer-readable recording medium may be any kind of media, such as a magnetic recording device, an optical disk, a magneto-optical recording medium, and semiconductor memory.

Processing means may be configured through execution of a predetermined program on a computer or at least some of the processing specifics thereof may be embodied in hardware.

The present invention is defined by the appended claims. It will be appreciated that modifications may be made as appropriate without departing from the scope of the appended claims.

[INDUSTRIAL APPLICABILITY]

As would be apparent from the result of application to genome-wide association study described above, the secure computation techniques of the present invention are applicable to performing Fisher's exact test via secure computation while keeping information on contingency tables concealed in an analysis utilizing Fisher's exact test, for example, genome-wide association study, genome analysis, clinical research, social survey, academic study, analysis of experimental results, marketing research, statistical calculations, medical information analysis, customer information analysis, and sales analysis.

**Claims**

1. A Fisher's exact test calculation apparatus for performing Fisher's exact test for a plurality of 2x2 contingency tables, the apparatus comprising:

a selection unit (4) that is adapted to select 2x2 contingency tables for which a result of Fisher's exact test indicative of being significant will be possibly obtained from among the plurality of 2x2 contingency tables based on a parameter obtained in the course of calculations for determining the result of Fisher's exact test; and
a calculation unit (2) that is adapted to perform calculations for determining the result of Fisher's exact test for each of the selected 2x2 contingency tables,
wherein the selection unit (4) is adapted to perform selection of the contingency tables while keeping the frequencies in the plurality of contingency tables concealed via multi-party secure computation,
where a, b, c, and d represent frequencies in a 2x2 contingency table and T represents significance level, the parameter obtained in calculation in course of determining the result of Fisher's exact test is $\log(p_a)$ defined by the formula below, and
the selection unit (4) is adapted to select 2x2 contingency tables with $\log(p_a)<\log(T)$ or, alternatively, $\log(p_a)\leq\log(T)$,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$
$$- \sum_{j=1}^{b} \log j - \sum_{j=1}^{c} \log j - \sum_{j=1}^{d} \log j$$

the plurality of contingency tables are a plurality of 2x2 contingency tables i (i=1, 2, ..., m), where m is a positive integer; the frequencies in the contingency table i are represented as $a_i$, $b_i$, $c_i$, $d_i$; information generated by concealing $a_i$, $b_i$, $c_i$, $d_i$, respectively, by using a multi-party encryption technique enabling multi-party secure computation of magnitude comparison, determination of equality, addition/subtraction, and multiplication, while concealing input and output, is represented as $E(a_i)$, $E(b_i)$, $E(c_i)$, $E(d_i)$; and information indicating whether the contingency table i is a 2x2 contingency table for which a result of Fisher's exact test indicative of being significant will be possibly obtained or not is represented as $X_i'$, wherein secure computation of magnitude comparison and determination of equality can determine cipher text $E(z)$ for the result of magnitude comparison or determination of equality by using $E(x)$ and y as the input without decrypting $E(x)$, and wherein secure computation of multiplication can determine $E(xy)$ from $E(x)$ and y as the input without decrypting $E(x)$,
the selection unit (4) is adapted to securely compute $E(X_i')$ from $E(a_i)$, $E(b_i)$, $E(C_i)$, $E(d_i)$ based on $E(\log(p_a))$ by secure computation of magnitude comparisons with input $E(\log(p_a))$ and $\log(T)$ so as to determine m sets, $(E(a_1), E(b_1), E(c_1), E(d_1), E(X_1'))$, $(E(a_2), E(b_2), E(C_2), E(d_2), E(X_2'))$, ..., $(E(a_m), E(b_m), E(c_m), E(d_m), E(X_m'))$, and shuffle an order of the m sets, $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$, while concealing the shuffled order, decrypt $E(X_i')$, and select 2x2 contingency tables for which a result of Fisher's exact test indicating that a result of the decryption is significant will be possibly obtained, wherein the selection unit (4) is adapted to calculate $E(\log(p_a))$ based on precomputed

values $\sum_{j=1}^{k} \log j$ for k=0, 1, ..., n, by, for each summand in the formula for log($p_a$) above, using secure computation of determining equality which returns E(e)=E(1) if r=k and E(e)=E(0) otherwise, where r is the upper limit of the running index j for the respective summand, and calculating $E(e \sum_{j=1}^{k} \log j)$ for each k=0, 1, ..., n from E(e) and $\sum_{j=1}^{k} \log j$ by secure computation of multiplication, and adding the obtained results for all k=0, 1, ..., n while keeping them encrypted to obtain cipher text E(S) of the respective summand, where S is the respective summand, and adding the cipher texts of the summands in the formula for log($p_a$) above while keeping them encrypted.

2. A Fisher's exact test calculation apparatus for performing Fisher's exact test for a plurality of 2x2 contingency tables, the apparatus comprising:

a selection unit (4) that is adapted to select 2x2 contingency tables for which a result of Fisher's exact test indicative of being significant will be possibly obtained from among the plurality of 2x2 contingency tables based on a parameter obtained in the course of calculations for determining the result of Fisher's exact test; and
a calculation unit (2) that is adapted to perform calculations for determining the result of Fisher's exact test for each of the selected 2x2 contingency tables,
wherein the selection unit (4) is adapted to perform selection of the contingency tables while keeping the frequencies in the plurality of contingency tables concealed via multi-party secure computation,
where a, b, c, and d represent frequencies in a 2x2 contingency table and T represents significance level, the parameter obtained in calculation in course of determining the result of Fisher's exact test is log($p_a$) defined by the formula below, and
the selection unit (4) is adapted to select 2x2 contingency tables with log($p_a$)<log(T) or, alternatively, log($p_a$)≤log(T),

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$
$$- \sum_{j=1}^{b} \log j - \sum_{j=1}^{c} \log j - \sum_{j=1}^{d} \log j$$

the plurality of contingency tables are a plurality of 2x2 contingency tables i (i=1, 2, ..., m), where m is a positive integer; the frequencies in the contingency table i are represented as $a_i$, $b_i$, $c_i$, $d_i$; information generated by concealing $a_i$, $b_i$, $c_i$, $d_i$, respectively, by using a multi-party encryption technique enabling multi-party secure computation of magnitude comparison, determination of equality, addition/subtraction, and multiplication, while concealing input and output, is represented as $E(a_i)$, $E(b_i)$, $E(c_i)$, $E(d_i)$, respectively; information indicating whether the contingency table i is a 2x2 contingency table for which a result of Fisher's exact test indicative of being significant will be possibly obtained or not is represented as $X_i'$, wherein secure computation of magnitude comparison and determination of equality can determine cipher text E(z) for the result of magnitude comparison or determination of equality by using E(x) and y as the input without decrypting E(x), and wherein secure computation of multiplication can determine E(xy) from E(x) and y as the input without decrypting E(x); and U is a positive integer,
the selection unit (4) is adapted to securely compute $E(X_i')$ from $E(a_i)$, $E(b_i)$, $E(c_i)$, $E(d_i)$ based on $E(\log(p_a))$ by secure computation of magnitude comparisons with input $E(\log(p_a))$ and log(T) so as to determine m sets, $(E(a_1), E(b_1), E(c_1), E(d_1), E(X_1'))$, $(E(a_2), E(b_2), E(C_2), E(d_2), E(X_2'))$, ..., $(E(a_m), E(b_m), E(c_m), E(d_m), E(X_m'))$, sort the m sets, $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$, while concealing $X_i'$ such that the information indicating whether the contingency table i is a 2x2 contingency table for which a result of Fisher's exact test indicative of being significant will be possibly obtained or not is located at a top or an end, and select U sets from the top or the end of the m sets after being sorted, wherein the selection unit (4) is adapted to calculate $E(\log(p_a))$ based on precomputed values $\sum_{j=1}^{k} \log j$ for k=0, 1, ..., n, by, for each summand in the formula for log($p_a$) above, using secure computation of determining equality which returns E(e)=E(1) if r=k and E(e)=E(0) otherwise, where r is the upper limit of the running index j for the respective summand, and calculating $E(e^{\sum_{j=1}^{k} \log j})$ for each k=0, 1, ..., n from E(e) and $\sum_{j=1}^{k} \log j$ by secure computation of multiplication, and adding the obtained results for all k=0, 1, ..., n while keeping them encrypted to obtain cipher text E(S) of the respective summand, where S is the respective summand, and adding the cipher texts of the summands in the formula for log($p_a$) above while keeping them encrypted.

3. A Fisher's exact test calculation apparatus for performing Fisher's exact test for a plurality of 2x2 contingency tables, the apparatus comprising:

a selection unit (4) that is adapted to select 2x2 contingency tables for which a result of Fisher's exact test indicative of being significant will be possibly obtained from among the plurality of 2x2 contingency tables based on a parameter obtained in the course of calculations for determining the result of Fisher's exact test; and

a calculation unit (2) that is adapted to perform calculations for determining the result of Fisher's exact test for each of the selected 2x2 contingency tables,

wherein the selection unit (4) is adapted to perform selection of the contingency tables while keeping the frequencies in the plurality of contingency tables concealed via multi-party secure computation,

where a, b, c, and d represent frequencies in a 2x2 contingency table and T represents significance level, the parameter obtained in calculation in course of determining the result of Fisher's exact test is log($p_a$) defined by the formula below, and

the selection unit (4) is adapted to select 2x2 contingency tables with log($p_a$)<log(T) or, alternatively, log($p_a$)≤log(T),

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$

$$- \sum_{j=1}^{b} \log j - \sum_{j=1}^{c} \log j - \sum_{j=1}^{d} \log j$$

the plurality of contingency tables are a plurality of 2x2 contingency tables i (i=1, 2, ..., m), where m is a positive integer; the frequencies in the contingency table i are represented as $a_i$, $b_i$, $c_i$, $d_i$; information generated by concealing $a_i$, $b_i$, $c_i$, $d_i$, respectively, by using a multi-party encryption technique enabling multi-party secure computation of magnitude comparison, determination of equality, addition/subtraction, and multiplication, while concealing input and output, is represented as E($a_i$), E($b_i$), E($c_i$), E($d_i$), respectively; and information indicating whether the contingency table i is a 2x2 contingency table for which a result of Fisher's exact test indicative of being significant will be possibly obtained or not is represented as $X_i'$, wherein secure computation of magnitude comparison and determination of equality can determine cipher text E(z) for the result of magnitude comparison or determination of equality by using E(x) and y as the input without decrypting E(x), and wherein secure computation of multiplication can determine E(xy) from E(x) and y as the input without decrypting E(x),

the selection unit (4) is adapted to securely compute E($X_i'$) from E($a_i$), E($b_i$), E($c_i$), E($d_i$) based on E(log($p_a$)) by secure computation of magnitude comparisons with input E(log($p_a$)) and log(T) so as to determine m sets, (E($a_1$), E($b_1$), E($c_1$), E($d_1$), E($X_1'$)), (E($a_2$), E($b_2$), E($C_2$), E($d_2$), E($X_2'$)), ..., (E($a_m$), E($b_m$), E($c_m$), E($d_m$), E($X_m'$)), and if $X_i'$ is information that represents not being a 2x2 contingency table for which a result of Fisher's exact test indicative of being significant will be possibly obtained, FALSE$_i'$, for at least one set of the m sets, (E($a_1$), E($b_1$), E($c_1$), E($d_1$), E($X_1'$)), (E($a_2$), E($b_2$), E($C_2$), E($d_2$), E($X_2'$)), ..., (E($a_m$), E($b_m$), E($c_m$), E($d_m$), E($X_m'$)), probabilistically replace that $X_i'$ with information that represents being a 2x2 contingency table for which a result of Fisher's exact test indicative of being significant will be possibly obtained, TRUE$_i'$, while concealing the $X_i'$, by preparing m pieces of data E($Y_1'$), E($Y_2'$), ..., E($Y_m'$) for which TRUE' or FALSE' is probabilistically concealed in advance, and by, while concealing $X_i'$ and $Y_i'$, calculating $Z_i'$=TRUE' if $X_i'$ represents TRUE$_i'$ or $Y_i'$ represents TRUE$_i'$, and $Z_i'$=FALSE$_i'$ otherwise, shuffle the order of the m sets, (E($a_1$), E($b_1$), E($c_1$), E($d_1$), E($X_1'$)), (E($a_2$), E($b_2$), E($C_2$), E($d_2$), E($X_2'$)), ..., (E($a_m$), E($b_m$), E($c_m$), E($d_m$), E($X_m'$)), after the replacement while concealing the shuffled order, decrypt $E(X_i')$, and select summary tables for which a result of Fisher's exact test indicating that a result of the decryption is significant will be possibly obtained, wherein the selection unit (4) is adapted to calculate $E(\log(p_a))$ based on precomputed values $\sum_{j=1}^{k}\log j$ for k=0, 1, ..., n, by, for each summand in the formula for $\log(p_a)$ above, using secure computation of determining equality which returns E(e)=E(1) if r=k and E(e)=E(0) otherwise, where r is the upper limit of the running index j for the respective summand, and calculating $E(e\sum_{j=1}^{k}\log j)$ for each k=0, 1, ..., n from E(e) and $\sum_{j=1}^{k}\log j$ by secure computation of multiplication, and adding the obtained results for all k=0, 1, ..., n while keeping them encrypted to obtain cipher text E(S) of the respective summand, where S is the respective summand, and adding the cipher texts of the summands in the formula for $\log(p_a)$ above while keeping them encrypted.

4. A computer-implemented method of performing Fisher's exact test for a plurality of 2x2 contingency tables, the method comprising:

   a selection step (S4) in which a selection unit (4) selects 2x2 contingency tables for which a result of Fisher's exact test indicative of being significant will be possibly obtained from among the plurality of 2x2 contingency tables based on a parameter obtained in the course of calculations for determining the result of Fisher's exact test; and
   a calculation step (S2) in which a calculation unit (2) performs calculations for determining the result of Fisher's exact test for each of the selected 2x2 contingency tables,
   wherein the selection unit (4) performs selection of the contingency tables while keeping the frequencies in the plurality of contingency tables concealed via multi-party secure computation,
   where a, b, c, and d represent frequencies in a 2x2 contingency table and T represents significance level, the parameter obtained in calculation in course of determining the result of Fisher's exact test is $\log(p_a)$ defined by the formula below, and
   the selection unit (4) selects 2x2 contingency tables with $\log(p_a)<\log(T)$ or, alternatively, $\log(p_a)\leq\log(T)$,

$$\log p_a = \sum_{j=1}^{a+b}\log j + \sum_{j=1}^{c+d}\log j + \sum_{j=1}^{a+c}\log j + \sum_{j=1}^{b+d}\log j - \sum_{j=1}^{n}\log j - \sum_{j=1}^{a}\log j$$
$$- \sum_{j=1}^{b}\log j - \sum_{j=1}^{c}\log j - \sum_{j=1}^{d}\log j$$

   the plurality of contingency tables are a plurality of 2x2 contingency tables i (i=1, 2, ..., m), where m is a positive integer; the frequencies in the contingency table i are represented as $a_i$, $b_i$, $c_i$, $d_i$; information generated by concealing $a_i$, $b_i$, $c_i$, $d_i$, respectively, by using a multi-party encryption technique enabling multi-party secure computation of magnitude comparison, determination of equality, addition/subtraction, and multiplication, while concealing input and output, is represented as $E(a_i)$, $E(b_i)$, $E(c_i)$, $E(d_i)$, respectively; and information indicating whether the contingency table i is a 2x2 contingency table for which a result of Fisher's exact test indicative of being significant will be possibly obtained or not is represented as $X_i'$, wherein secure computation of magnitude comparison and determination of equality can determine cipher text E(z) for the result of magnitude comparison or determination of equality by using E(x) and y as the input without decrypting E(x), and wherein secure computation of multiplication can determine E(xy) from E(x) and y as the input without decrypting E(x),
   the selection unit (4) securely computes $E(X_i')$ from $E(a_i)$, $E(b_i)$, $E(C_i)$, $E(d_i)$ based on $E(\log(p_a))$ by secure computation of magnitude comparisons with input $E(\log(p_a))$ and log(T) so as to determine m sets, $(E(a_1), E(b_1), E(c_1), E(d_1), E(X_1')), (E(a_2), E(b_2), E(C_2), E(d_2), E(X_2')), ..., (E(a_m), E(b_m), E(c_m), E(d_m), E(X_m'))$, and shuffles an order of the m sets, $(E(a_1), E(b_1), E(c_1), E(d_1), E(X_1')), (E(a_2), E(b_2), E(c_2), E(d_2), E(X_2')), ..., (E(a_m), E(b_m), E(c_m), E(d_m), E(X_m'))$, while concealing the shuffled order, decrypts $E(X_i')$, and selects 2x2 contingency tables for which a result of Fisher's exact test indicating that a result of the decryption is significant will be possibly obtained, wherein the selection unit (4) calculates $E(\log(p_a))$ based on precomputed values $\sum_{j=1}^{k}\log j$ for k=0, 1, ..., n, by, for each summand in the formula for $\log(p_a)$ above, using secure computation of determining equality which returns E(e)=E(1) if r=k and E(e)=E(0) otherwise, where r is the upper limit of the running index j for the respective summand, and calculating $E(e^{\sum_{j=1}^{k} \log j})$ for each k=0, 1, ..., n from E(e) and $\sum_{j=1}^{k} \log j$ by secure computation of multiplication, and adding the obtained results for all k=0, 1, ..., n while keeping them encrypted to obtain cipher text E(S) of the respective summand, where S is the respective summand, and adding the cipher texts of the summands in the formula for log($p_a$) above while keeping them encrypted.

5. A computer-implemented method of performing Fisher's exact test for a plurality of 2x2 contingency tables, the method comprising:

a selection step (S4) in which a selection unit (4) selects 2x2 contingency tables for which a result of Fisher's exact test indicative of being significant will be possibly obtained from among the plurality of 2x2 contingency tables based on a parameter obtained in the course of calculations for determining the result of Fisher's exact test; and
a calculation step (S2) in which a calculation unit (2) performs calculations for determining the result of Fisher's exact test for each of the selected 2x2 contingency tables,
wherein the selection unit (4) performs selection of the contingency tables while keeping the frequencies in the plurality of contingency tables concealed via multi-party secure computation,
where a, b, c, and d represent frequencies in a 2x2 contingency table and T represents significance level, the parameter obtained in calculation in course of determining the result of Fisher's exact test is log($p_a$) defined by the formula below, and
the selection unit (4) selects 2x2 contingency tables with log($p_a$)<log(T) or, alternatively, log($p_a$)≤log(T),

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$
$$- \sum_{j=1}^{b} \log j - \sum_{j=1}^{c} \log j - \sum_{j=1}^{d} \log j$$

the plurality of contingency tables are a plurality of 2x2 contingency tables i (i=1, 2, ..., m), where m is a positive integer; the frequencies in the contingency table i are represented as $a_i$, $b_i$, $c_i$, $d_i$; information generated by concealing $a_i$, $b_i$, $c_i$, $d_i$, respectively, by using a multi-party encryption technique enabling multi-party secure computation of magnitude comparison, determination of equality, addition/subtraction, and multiplication, while concealing input and output, is represented as E($a_i$), E($b_i$), E($c_i$), E($d_i$), respectively; information indicating whether the contingency table i is a 2x2 contingency table for which a result of Fisher's exact test indicative of being significant will be possibly obtained or not is represented as $X_i'$, wherein secure computation of magnitude comparison and determination of equality can determine cipher text E(z) for the result of magnitude comparison or determination of equality by using E(x) and y as the input without decrypting E(x), and wherein secure computation of multiplication can determine E(xy) from E(x) and y as the input without decrypting E(x); and U is a positive integer,
the selection unit (4) securely computes E($X_i'$) from E($a_i$), E($b_i$), E($c_i$), E($d_i$) based on E(log($p_a$)) by secure computation of magnitude comparisons with input E(log($p_a$)) and log(T) so as to determine m sets, (E($a_1$), E($b_1$), E($c_1$), E($d_1$), E($X_1'$)), (E($a_2$), E($b_2$), E($C_2$), E($d_2$), E($X_2'$)), ..., (E($a_m$), E($b_m$), E($c_m$), E($d_m$), E($X_m'$)), sorts the m sets, (E($a_1$), E($b_1$), E($c_1$), E($d_1$), E($X_1'$)), (E($a_2$), E($b_2$), E($c_2$), E($d_2$), E($X_2'$)), ..., (E($a_m$), E($b_m$), E($c_m$), E($d_m$), E($X_m'$)), while concealing $X_i'$ such that the information indicating whether the contingency table i is a 2x2 contingency table for which a result of Fisher's exact test indicative of being significant will be possibly obtained or not is located at a top or an end, and selects U sets from the top or the end of the m sets after being sorted,
wherein the selection unit (4) calculates E(log($p_a$)) based on precomputed values $\sum_{j=1}^{k} \log j$ for k=0, 1, ..., n, by, for each summand in the formula for log($p_a$) above, using secure computation of determining equality which returns E(e)=E(1) if r=k and E(e)=E(0) otherwise, where r is the upper limit of the running index j for the respective summand, and calculating $E(e^{\sum_{j=1}^{k} \log j})$ for each k=0, 1, ..., n from E(e) and $\sum_{j=1}^{k} \log j$ by secure computation of multiplication, and adding the obtained results for all k=0, 1, ..., n while keeping them encrypted to obtain cipher text E(S) of the respective summand, where S is the respective summand, and adding the cipher texts of the summands in the formula for log($p_a$) above while keeping them encrypted.

**6.** A computer-implemented method of performing Fisher's exact test for a plurality of 2x2 contingency tables, the method comprising:

a selection step (S4) in which a selection unit (4) selects 2x2 contingency tables for which a result of Fisher's exact test indicative of being significant will be possibly obtained from among the plurality of 2x2 contingency tables based on a parameter obtained in the course of calculations for determining the result of Fisher's exact test; and

a calculation step (S2) in which a calculation unit (2) performs calculations for determining the result of Fisher's exact test for each of the selected 2x2 contingency tables,

wherein the selection unit 2- (4) performs selection of the contingency tables while keeping the frequencies in the plurality of contingency tables concealed via multi-party secure computation,

where a, b, c, and d represent frequencies in a 2x2 contingency table and T represents significance level, the parameter obtained in calculation in course of determining the result of Fisher's exact test is log($p_a$) defined by the formula below, and

the selection unit (4) selects 2x2 contingency tables with log($p_a$)<log(T) or, alternatively, log($p_a$)≤log(T),

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$
$$- \sum_{j=1}^{b} \log j - \sum_{j=1}^{c} \log j - \sum_{j=1}^{d} \log j$$

the plurality of contingency tables are a plurality of 2x2 contingency tables i (i=1, 2, ..., m), where m is a positive integer; the frequencies in the contingency table i are represented as $a_i$, $b_i$, $c_i$, $d_i$; information generated by concealing $a_i$, $b_i$, $c_i$, $d_i$, respectively, by using a multi-party encryption technique enabling multi-party secure computation of magnitude comparison, determination of equality, addition/subtraction, and multiplication, while concealing input and output, is represented as E($a_i$), E($b_i$), E($c_i$), E($d_i$), respectively; and information indicating whether the contingency table i is a 2x2 contingency table for which a result of Fisher's exact test indicative of being significant will be possibly obtained or not is represented as $X_i'$, wherein secure computation of magnitude comparison and determination of equality can determine cipher text E(z) for the result of magnitude comparison or determination of equality by using E(x) and y as the input without decrypting E(x), and wherein secure computation of multiplication can determine E(xy) from E(x) and y as the input without decrypting E(x),

the selection unit (4) securely computes E($X_i'$) from E($a_i$), E($b_i$), E($c_i$), E($d_i$) based on E(log($p_a$)) by secure computation of magnitude comparisons with input E(log($p_a$)) and log(T) so as to determine m sets, (E($a_1$), E($b_1$), E($c_1$), E($d_1$), E($X_1'$)), (E($a_2$), E($b_2$), E($C_2$), E($d_2$), E($X_2'$)), ..., (E($a_m$), E($b_m$), E($c_m$), E($d_m$), E($X_m'$)), and if $X_i'$ is information that represents not being a 2x2 contingency table for which a result of Fisher's exact test indicative of being significant will be possibly obtained, FALSE$_i'$, for at least one set of the m sets, (E($a_1$), E($b_1$), E($c_1$), E($d_1$), E($X_1'$)), (E($a_2$), E($b_2$), E($C_2$), E($d_2$), E($X_2'$)), ..., (E($a_m$), E($b_m$), E($c_m$), E($d_m$), E($X_m'$)), probabilistically replaces that $X_i'$ with information that represents being a 2x2 contingency table for which a result of Fisher's exact test indicative of being significant will be possibly obtained, TRUE$_i'$, while concealing the $X_i'$, by preparing m pieces of data E($Y_1'$), E($Y_2'$), ..., E($Y_m'$) for which TRUE' or FALSE' is probabilistically concealed in advance, and by, while concealing $X_i'$ and $Y_i'$, calculating $Z_i'$=TRUE' if $X_i'$ represents TRUE$_i'$ or $Y_i'$ represents TRUE$_i'$, and $Z_i'$=FALSE$_i'$ otherwise, shuffles the order of the m sets, (E($a_1$), E($b_1$), E($c_1$), E($d_1$), E($X_1'$)), (E($a_2$), E($b_2$), E($c_2$), E($d_2$), E($X_2'$)), ..., (E($a_m$), E($b_m$), E($c_m$), E($d_m$), E($X_m'$)), after the replacement while concealing the shuffled order, decrypts E($X_i'$), and selects summary tables for which a result of Fisher's exact test indicating that a result of the decryption is significant will be possibly obtained, wherein the selection unit (4) calculates E(log($p_a$))

based on precomputed values $\sum_{j=1}^{k} \log j$ for k=0, 1, ..., n, by, for each summand in the formula for log($p_a$) above, using secure computation of determining equality which returns E(e)=E(1) if r=k and E(e)=E(0) otherwise, where r is the upper limit of the running index j for the respective summand, and calculating $E(e^{\sum_{j=1}^{k} \log j})$ for each k=0, 1, ..., n from E(e) and $\sum_{j=1}^{k} \log j$ by secure computation of multiplication, and adding the obtained results for all k=0, 1, ..., n while keeping them encrypted to obtain cipher text E(S) of the respective summand, where S is the respective summand, and adding the cipher texts of the summands in the formula for log(p$_a$) above while keeping them encrypted.

7. A program for causing a computer to function as the units (2, 4) of the Fisher's exact test calculation apparatus according to any one of Claims 1 to 3.

**Patentansprüche**

1. Vorrichtung zur Berechnung vom exakten Test nach Fisher zum Durchführen vom exakten Test nach Fisher für eine Vielzahl von 2x2-Kontingenztafeln, die Vorrichtung Folgendes umfassend:

eine Auswahleinheit (4), welche eingerichtet ist, um 2x2-Kontingenztafeln auszuwählen, für welche ein Ergebnis vom exakten Test nach Fisher, welches als signifikant bezeichnet ist, möglicherweise aus der Vielzahl von 2x2-Kontingenztafeln auf der Grundlage eines Parameters ermittelt wird, welcher im Verlauf von Berechnungen zum Bestimmen des Ergebnisses vom exakten Test nach Fisher ermittelt wurde; und
eine Berechnungseinheit (2), welche eingerichtet ist, um Berechnungen zum Bestimmen des Ergebnisses vom exakten Test nach Fisher für jede der ausgewählten 2x2-Kontingenztafeln durchzuführen,
wobei die Auswahleinheit (4) eingerichtet ist, um eine Auswahl der Kontingenztafeln durchzuführen, während die Frequenzen in der Vielzahl von Kontingenztafeln durch sichere verteilte Berechnung verborgen gehalten werden, wobei a, b, c und d Frequenzen in einer 2x2-Kontingenztafel repräsentieren und T einen Signifikanzlevel repräsentiert, der Parameter, welcher in einer Berechnung im Verlauf des Bestimmens des Ergebnisses vom exakten Test nach Fisher ermittelt wird, log(p$_a$) ist, wie durch die untenstehende Formel definiert, und
die Auswahleinheit (4) eingerichtet ist, um 2x2-Kontingenztafeln mit log(p$_a$) < log(T) oder alternativ mit log(p$_a$) ≤ log(T) auszuwählen,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$
$$- \sum_{j=1}^{b} \log j - \sum_{j=1}^{c} \log j - \sum_{j=1}^{d} \log j$$

die Vielzahl von Kontingenztafeln eine Vielzahl von 2x2-Kontingenztafeln i (i = 1, 2, ..., m) ist, wobei m eine positive ganze Zahl ist; die Frequenzen in der Kontingenztafel i als a$_i$, b$_i$, c$_i$, d$_i$ dargestellt sind; Informationen, welche durch Verbergen von a$_i$, b$_i$, c$_i$ bzw. d$_i$ unter Verwendung eines verteilten Verschlüsselungsverfahrens erzeugt werden, welches eine sichere verteilte Berechnung eines Größenvergleichs, einer Gleichheitsbestimmung, einer Addition/Subtraktion und einer Multiplikation ermöglicht, während Eingaben und Ausgaben verborgen werden, als E(a$_i$), E(b$_i$), E(c$_i$), E(d$_i$) dargestellt werden; und eine Information, welche angibt ob die Kontingenztafel i eine 2x2-Kontingenztafel ist, für welche ein Ergebnis vom exakten Test nach Fisher, welches als signifikant bezeichnet ist, möglicherweise ermittelt wird oder nicht, als X$_i$' dargestellt ist, wobei eine sichere Berechnung eines Größenvergleichs und einer Gleichheitsbestimmung einen Schlüsseltext E(z) für das Ergebnis des Größenvergleichs oder der Gleichheitsbestimmung unter Verwendung von E(x) und y als die Eingabe ohne Entschlüsseln von E(x) bestimmen kann und wobei eine sichere Berechnung der Multiplikation E(xy) aus E (x) und y als die Eingabe ohne Entschlüsseln von E(x) bestimmen kann,
die Auswahleinheit (4) eingerichtet ist, um E(X$_i$') aus E(a$_i$), E(b$_i$), E(c$_i$), E(d$_i$) auf der Grundlage von E(log(p$_a$)) durch sichere Berechnung von Größenvergleichen mit einer Eingabe E(log(p$_a$)) und log(T) sicher zu berechnen, um so m Mengen (E(a$_1$), E(b$_i$), E(c$_1$), E(d$_1$), E(X$_1$')), (E(a$_2$), E(b$_2$), E(c$_2$), E(d$_2$), E(X$_2$')), ..., (E(a$_m$), E(b$_m$), E(c$_m$), E(d$_m$), E(X$_m$')) zu bestimmen, und eine Reihenfolge der m Mengen (E(a$_1$), E(b$_i$), E(c$_1$), E(d$_i$), E(X$_1$')), (E(a$_2$), E(b$_2$), E(c$_2$), E(d$_2$), E(X$_2$')), ..., (E(a$_m$), E(b$_m$), E(c$_m$), E(d$_m$), E(X$_m$')) umzuordnen, während die umgeordnete Reihenfolge verborgen wird, $E(X_i')$ zu entschlüsseln und 2x2-Kontingenztafeln auszuwählen, für welche ein Ergebnis vom exakten Test nach Fisher, welches angibt, dass ein Ergebnis der Entschlüsselung signifikant ist, möglicherweise ermittelt wird, wobei die Auswahleinheit (4) eingerichtet ist, um $E(\log(p_a))$ auf der Grundlage vorberechneter Werte $\sum_{j=1}^{k} \log j$ für k = 0, 1, ..., n zu berechnen, indem für jeden Summanden in der obenstehenden Formel für $\log(p_a)$ eine sichere Berechnung einer Gleichheitsbestimmung verwendet wird, welche $E(e) = E(1)$, wenn r = k, und andernfalls $E(e) = E(0)$ ergibt, wobei r die Obergrenze des laufenden Indexes j für den jeweiligen Summanden ist, und $E(\sum_{j=1}^{k} \log j)$ für jedes k = 0, 1, ..., n aus $E(e)$ und $\sum_{j=1}^{k} \log j$ durch sichere Berechnung einer Multiplikation berechnet wird, und die ermittelten Ergebnisse für alle k = 0, 1, ..., n, während sie verschlüsselt gehalten werden, addiert werden, um einen Schlüsseltext $E(S)$ des jeweiligen Summanden zu ermitteln, wobei S der jeweilige Summand ist, und die Schlüsseltexte der Summanden in der obenstehenden Formel für $\log(p_a)$ addiert werden, während sie verschlüsselt gehalten werden.

2. Vorrichtung zur Berechnung vom exakten Test nach Fisher zum Durchführen vom exakten Test nach Fisher für eine Vielzahl von 2x2-Kontingenztafeln, die Vorrichtung Folgendes umfassend:

eine Auswahleinheit (4), welche eingerichtet ist, um 2x2-Kontingenztafeln auszuwählen, für welche ein Ergebnis vom exakten Test nach Fisher, welches als signifikant bezeichnet ist, möglicherweise aus der Vielzahl von 2x2-Kontingenztafeln auf der Grundlage eines Parameters ermittelt wird, welcher im Verlauf von Berechnungen zum Bestimmen des Ergebnisses vom exakten Test nach Fisher ermittelt wurde; und
eine Berechnungseinheit (2), welche eingerichtet ist, um Berechnungen zum Bestimmen des Ergebnisses vom exakten Test nach Fisher für jede der ausgewählten 2x2-Kontingenztafeln durchzuführen, wobei die Auswahleinheit (4) eingerichtet ist, um eine Auswahl der Kontingenztafeln durchzuführen, während die Frequenzen in der Vielzahl von Kontingenztafeln durch sichere verteilte Berechnung verborgen gehalten werden, wobei a, b, c und d Frequenzen in einer 2x2-Kontingenztafel repräsentieren und T einen Signifikanzlevel repräsentiert, der Parameter, welcher in einer Berechnung im Verlauf des Bestimmens des Ergebnisses vom exakten Test nach Fisher ermittelt wird, $\log(p_a)$ ist, wie durch die untenstehende Formel definiert, und
die Auswahleinheit (4) eingerichtet ist, um 2x2-Kontingenztafeln mit $\log(p_a) < \log(T)$ oder alternativ mit $\log(p_a) \leq \log(T)$ auszuwählen,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$
$$- \sum_{j=1}^{b} \log j - \sum_{j=1}^{c} \log j - \sum_{j=1}^{d} \log j$$

die Vielzahl von Kontingenztafeln eine Vielzahl von 2x2-Kontingenztafeln i (i = 1, 2, ..., m) ist, wobei m eine positive ganze Zahl ist; die Frequenzen in der Kontingenztafel i als $a_i$, $b_i$, $c_i$, $d_i$ dargestellt sind; Informationen, welche durch Verbergen von $a_i$, $b_i$, $c_i$ bzw. $d_i$ unter Verwendung eines verteilten Verschlüsselungsverfahrens erzeugt werden, welches eine sichere verteilte Berechnung eines Größenvergleichs, einer Gleichheitsbestimmung, einer Addition/Subtraktion und einer Multiplikation ermöglicht, während Eingaben und Ausgaben verborgen werden, als $E(a_i)$, $E(b_i)$, $E(c_i)$ bzw. $E(d_i)$ dargestellt werden; eine Information, welche angibt ob die Kontingenztafel i eine 2x2-Kontingenztafel ist, für welche ein Ergebnis vom exakten Test nach Fisher, welches als signifikant bezeichnet ist, möglicherweise ermittelt wird oder nicht, als $X_i'$ dargestellt ist, wobei eine sichere Berechnung eines Größenvergleichs und einer Gleichheitsbestimmung einen Schlüsseltext $E(z)$ für das Ergebnis des Größenvergleichs oder der Gleichheitsbestimmung unter Verwendung von $E(x)$ und y als die Eingabe ohne Entschlüsseln von $E(x)$ bestimmen kann und wobei eine sichere Berechnung der Multiplikation $E(xy)$ aus $E(x)$ und y als die Eingabe ohne Entschlüsseln von $E(x)$ bestimmen kann; und U eine positive ganze Zahl ist, die Auswahleinheit (4) eingerichtet ist, um $E(X_i')$ aus $E(a_i)$, $E(b_i)$, $E(c_i)$, $E(d_i)$ auf der Grundlage von $E(\log(p_a))$ durch sichere Berechnung von Größenvergleichen mit einer Eingabe $E(\log(p_a))$ und $\log(T)$ sicher zu berechnen, um so m Mengen $(E(a_1), E(b_i), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$ zu bestimmen, die m Mengen $(E(a_1), E(b_i), E(c_1), E(d_1) E(X_1'))$, $(E(a_2), E(b_2), E(c_2), E(d_2))$,

E($X_2$')), ..., (E($a_m$), E($b_m$), E($c_m$), E($d_m$), E($X_m$')) zu sortieren, während $X_i$' verborgen wird, so dass die Information, welche angibt ob die Kontingenztafel i eine 2x2-Kontingenztafel ist, für welche ein Ergebnis vom exakten Test nach Fisher, welches als signifikant bezeichnet ist, möglicherweise ermittelt wird oder nicht, oben oder am Ende lokalisiert ist, und U Mengen von oben oder vom Ende der m Mengen auszuwählen, nachdem sie sortiert sind, wobei die Auswahleinheit (4) eingerichtet ist, um E($\log(p_a)$) auf der Grundlage vorberechneter Werte $\sum_{j=1}^{k} \log j$ für k = 0, 1, ..., n zu berechnen, indem für jeden Summanden in der obenstehenden Formel für $\log(p_a)$ eine sichere Berechnung einer Gleichheitsbestimmung verwendet wird, welche E(e) = E(1), wenn r = k, und andernfalls E(e) = E(0) ergibt, wobei r die Obergrenze des laufenden Indexes j für den jeweiligen Summanden ist, und $E(\sum_{j=1}^{k} \log j)$ für jedes k = 0, 1, ..., n aus E(e) und $\sum_{j=1}^{k} \log j$ durch sichere Berechnung einer Multiplikation berechnet wird, und die ermittelten Ergebnisse für alle k = 0, 1, ..., n addiert werden, während sie verschlüsselt gehalten werden, um einen Schlüsseltext E(S) des jeweiligen Summanden zu ermitteln, wobei S der jeweilige Summand ist, und die Schlüsseltexte der Summanden in der obenstehenden Formel für $\log(p_a)$ addiert werden, während sie verschlüsselt gehalten werden.

3.  Vorrichtung zur Berechnung vom exakten Test nach Fisher zum Durchführen vom exakten Test nach Fisher für eine Vielzahl von 2x2-Kontingenztafeln, die Vorrichtung Folgendes umfassend:

    eine Auswahleinheit (4), welche eingerichtet ist, um 2x2-Kontingenztafeln auszuwählen, für welche ein Ergebnis vom exakten Test nach Fisher, welches als signifikant bezeichnet ist, möglicherweise aus der Vielzahl von 2x2-Kontingenztafeln auf der Grundlage eines Parameters ermittelt wird, welcher im Verlauf von Berechnungen zum Bestimmen des Ergebnisses vom exakten Test nach Fisher ermittelt wurde; und
    eine Berechnungseinheit (2), welche eingerichtet ist, um Berechnungen zum Bestimmen des Ergebnisses vom exakten Test nach Fisher für jede der ausgewählten 2x2-Kontingenztafeln durchzuführen,
    wobei die Auswahleinheit (4) eingerichtet ist, um eine Auswahl der Kontingenztafeln durchzuführen, während die Frequenzen in der Vielzahl von Kontingenztafeln durch sichere verteilte Berechnung verborgen gehalten werden, wobei a, b, c und d Frequenzen in einer 2x2-Kontingenztafel repräsentieren und T einen Signifikanzlevel repräsentiert, der Parameter, welcher in einer Berechnung im Verlauf des Bestimmens des Ergebnisses vom exakten Test nach Fisher ermittelt wird, $\log(p_a)$ ist, wie durch die untenstehende Formel definiert, und
    die Auswahleinheit (4) eingerichtet ist, um 2x2-Kontingenztafeln mit $\log(p_a) < \log(T)$ oder alternativ mit $\log(p_a) \leq \log(T)$ auszuwählen,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$
$$- \sum_{j=1}^{b} \log j - \sum_{j=1}^{c} \log j - \sum_{j=1}^{d} \log j$$

die Vielzahl von Kontingenztafeln eine Vielzahl von 2x2-Kontingenztafeln i (i = 1, 2, ..., m) ist, wobei m eine positive ganze Zahl ist; die Frequenzen in der Kontingenztafel i als $a_i$, $b_i$, $c_i$, $d_i$ dargestellt sind; Informationen, welche durch Verbergen von $a_i$, $b_i$, $c_i$ bzw. $d_i$ unter Verwendung eines verteilten Verschlüsselungsverfahrens erzeugt werden, welches eine sichere verteilte Berechnung eines Größenvergleichs, einer Gleichheitsbestimmung, einer Addition/Subtraktion und einer Multiplikation ermöglicht, während Eingaben und Ausgaben verborgen werden, als E($a_i$), E($b_i$), E($c_i$) bzw. E($d_i$) dargestellt werden; eine Information, welche angibt ob die Kontingenztafel i eine 2x2-Kontingenztafel ist, für welche ein Ergebnis vom exakten Test nach Fisher, welches als signifikant bezeichnet ist, möglicherweise ermittelt wird oder nicht, als $X_i$' dargestellt ist, wobei eine sichere Berechnung eines Größenvergleichs und einer Gleichheitsbestimmung einen Schlüsseltext E(z) für das Ergebnis des Größenvergleichs oder der Gleichheitsbestimmung unter Verwendung von E(x) und y als die Eingabe ohne Entschlüsseln von E(x) bestimmen kann und wobei eine sichere Berechnung der Multiplikation E(xy) aus E(x) und y als die Eingabe ohne Entschlüsseln von E(x) bestimmen kann,
die Auswahleinheit (4) eingerichtet ist, um E($X_i$') aus E($a_i$), E($b_i$), E($c_i$), E($d_i$) auf der Grundlage von E($\log(p_a)$) durch sichere Berechnung von Größenvergleichen mit einer Eingabe E($\log(p_a)$) und $\log(T)$ sicher zu berechnen, um so m Mengen (E($a_1$), E($b_1$), E($c_1$), E($d_1$) E($X_1$')), (E($a_2$), E($b_2$), E($c_2$), E($d_2$), E($X_2$')), ..., (E($a_m$), E($b_m$),

$E(c_m)$, $E(d_m)$, $E(X_m')$) zu bestimmen, und, wenn $X_i'$ eine Information, welche darstellt, dass sie keine 2x2-Kontingenztafel ist, für welche ein Ergebnis vom exakten Test nach Fisher, das als signifikant bezeichnet ist, möglicherweise ermittelt wird, $FALSE_i'$ für mindestens eine Menge der m Mengen, $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$ ist, dieses $X_i'$ probabilistisch durch eine Information, welche darstellt, dass sie eine 2x2-Kontingenztafel ist, für welche ein Ergebnis vom exakten Test nach Fisher, das als signifikant bezeichnet ist, möglicherweise ermittelt wird, $TRUE_i'$ zu ersetzen, während das $X_i'$ verborgen wird, indem m Datenstücke $E(Y_1')$, $E(Y_2')$, ..., $E(Y_m')$, für welche im Voraus TRUE' oder FALSE' probabilistisch verborgen wurde, vorbereitet werden und indem, während $X_i'$ und $Y_i'$ verborgen sind, $Z_i'$ = TRUE', wenn $X_i'$ $TRUE_i'$ repräsentiert oder $Y_i'$ $TRUE_i'$ repräsentiert, und andernfalls $Z_i'$ = $FALSE_i'$ berechnet wird, die Reihenfolge der m Mengen $(E(a_1), E(b_1),E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$ nach dem Ersetzen umzuordnen, während die umgeordnete Reihenfolge verborgen wird, $E(X_i')$ zu entschlüsseln und Summentafeln auszuwählen, für welche ein Ergebnis vom exakten Test nach Fisher, welches angibt, dass ein Ergebnis der Entschlüsselung signifikant ist, möglicherweise ermittelt wird, wobei die Auswahleinheit (4) eingerichtet ist, um $E(\log(p_a))$ auf der Grundlage vorberechneter Werte $\sum_{j=1}^{k} \log j$ für k = 0, 1, ..., n zu berechnen, indem für jeden Summanden in der obenstehenden Formel für $\log(p_a)$ eine sichere Berechnung einer Gleichheitsbestimmung verwendet wird, welche E(e) = E(1), wenn r = k, und andernfalls E(e) = E(0) ergibt, wobei r die Obergrenze des laufenden Indexes j für den jeweiligen Summanden ist, und $E(\sum_{j=1}^{k} \log j)$ für jedes k = 0, 1, ..., n aus E(e) und $\sum_{j=1}^{k} \log j$ durch sichere Berechnung einer Multiplikation berechnet wird, und die ermittelten Ergebnisse für alle k = 0, 1, ..., n addiert werden, während sie verschlüsselt gehalten werden, um einen Schlüsseltext E(S) des jeweiligen Summanden zu ermitteln, wobei S der jeweilige Summand ist, und die Schlüsseltexte der Summanden in der obenstehenden Formel für $\log(p_a)$ addiert werden, während sie verschlüsselt gehalten werden.

4. Computerimplementiertes Verfahren zum Durchführen vom exakten Test nach Fisher für eine Vielzahl von 2x2-Kontingenztafeln, das Verfahren Folgendes umfassend:

   einen Auswahlschritt (S4), in welchem eine Auswahleinheit (4) 2x2-Kontingenztafeln auswählt, für welche ein Ergebnis vom exakten Test nach Fisher, welches als signifikant bezeichnet ist, möglicherweise aus der Vielzahl von 2x2-Kontingenztafeln auf der Grundlage eines Parameters ermittelt wird, welcher im Verlauf von Berechnungen zum Bestimmen des Ergebnisses vom exakten Test nach Fisher ermittelt wurde; und
   einen Berechnungsschritt (S2), in welchem eine Berechnungseinheit (2) Berechnungen zum Bestimmen des Ergebnisses vom exakten Test nach Fisher für jede der ausgewählten 2x2-Kontingenztafeln durchführt,
   wobei die Auswahleinheit (4) eine Auswahl der Kontingenztafeln durchführt, während die Frequenzen in der Vielzahl von Kontingenztafeln durch sichere verteilte Berechnung verborgen gehalten werden,
   wobei a, b, c und d Frequenzen in einer 2x2-Kontingenztafel repräsentieren und T einen Signifikanzlevel repräsentiert, der Parameter, welcher in einer Berechnung im Verlauf des Bestimmens des Ergebnisses vom exakten Test nach Fisher ermittelt wird, $\log(p_a)$ ist, wie durch die untenstehende Formel definiert, und die Auswahleinheit (4) 2x2-Kontingenztafeln mit $\log(p_a) < \log(T)$ oder alternativ mit $\log(p_a) \leq \log(T)$ auswählt,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j - \sum_{j=1}^{c} \log j - \sum_{j=1}^{d} \log j$$

die Vielzahl von Kontingenztafeln eine Vielzahl von 2x2-Kontingenztafeln i (i = 1, 2, ..., m) ist, wobei m eine positive ganze Zahl ist; die Frequenzen in der Kontingenztafel i als $a_i$, $b_i$, $c_i$, $d_i$ dargestellt sind; Informationen, welche durch Verbergen von $a_i$, $b_i$, $c_i$ bzw. $d_i$ unter Verwendung eines verteilten Verschlüsselungsverfahrens erzeugt werden, welches eine sichere verteilte Berechnung eines Größenvergleichs, einer Gleichheitsbestimmung, einer Addition/Subtraktion und einer Multiplikation ermöglicht, während Eingaben und Ausgaben verborgen werden, als $E(a_i)$, $E(b_i)$, $E(c_i)$ bzw. $E(d_i)$ dargestellt werden; eine Information, welche angibt ob die Kontingenztafel i eine 2x2-Kontingenztafel ist, für welche ein Ergebnis vom exakten Test nach Fisher, welches als signifikant bezeichnet ist, möglicherweise ermittelt wird oder nicht, als $X_i'$ dargestellt ist, wobei eine sichere Berechnung eines Größenvergleichs und einer Gleichheitsbestimmung einen Schlüsseltext $E(z)$ für das Ergebnis des Größenvergleichs oder der Gleichheitsbestimmung unter Verwendung von $E(x)$ und y als die Eingabe ohne Entschlüsseln von $E(x)$ bestimmen kann und wobei eine sichere Berechnung der Multiplikation $E(xy)$ aus $E(x)$ und y als die Eingabe ohne Entschlüsseln von $E(x)$ bestimmen kann,

die Auswahleinheit (4) $E(X_i')$ aus $E(a_i)$, $E(b_i)$, $E(c_i)$, $E(d_i)$ auf der Grundlage von $E(\log(p_a))$ durch sichere Berechnung von Größenvergleichen mit einer Eingabe $E(\log(p_a))$ und $\log(T)$ sicher berechnet, um so m Mengen $(E(a_1), E(b_1), E(c_1), E(d_1), E(X_1')), (E(a_2), E(b_2), E(c_2), E(d_2), E(X_2')), ..., (E(a_m), E(b_m), E(c_m), E(d_m), E(X_m'))$ zu bestimmen, und eine Reihenfolge der m Mengen $(E(a_1), E(b_1), E(c_1), E(d_1), E(X_1')), (E(a_2), E(b_2), E(c_2), E(d_2), E(X_2')), ..., (E(a_m), E(b_m), E(c_m), E(d_m), E(X_m'))$ umordnet, während die umgeordnete Reihenfolge verborgen wird, $E(X_i')$ entschlüsselt und 2x2-Kontingenztafeln auswählt, für welche ein Ergebnis vom exakten Test nach Fisher, welches angibt, dass ein Ergebnis der Entschlüsselung signifikant ist, möglicherweise ermittelt wird, wobei die Auswahleinheit (4) $E(\log(p_a))$ auf der Grundlage vorberechneter Werte $\sum_{j=1}^{k} \log j$ für k = 0, 1, ..., n berechnet, indem für jeden Summanden in der obenstehenden Formel für $\log(p_a)$ eine sichere Berechnung einer Gleichheitsbestimmung verwendet wird, welche $E(e) = E(1)$, wenn r = k, und andernfalls $E(e) = E(0)$ ergibt, wobei r die Obergrenze des laufenden Indexes j für den jeweiligen Summanden ist, und $E(\sum_{j=1}^{k} \log j)$ für jedes k = 0, 1, ..., n aus $E(e)$ und $\sum_{j=1}^{k} \log j$ durch sichere Berechnung einer Multiplikation berechnet wird, und die ermittelten Ergebnisse für alle k = 0, 1, ..., n addiert werden, während sie verschlüsselt gehalten werden, um einen Schlüsseltext $E(S)$ des jeweiligen Summanden zu ermitteln, wobei S der jeweilige Summand ist, und die Schlüsseltexte der Summanden in der obenstehenden Formel für $\log(p_a)$ addiert werden, während sie verschlüsselt gehalten werden.

5. Computerimplementiertes Verfahren zum Durchführen vom exakten Test nach Fisher für eine Vielzahl von 2x2-Kontingenztafeln, das Verfahren Folgendes umfassend:

einen Auswahlschritt (S4), in welchem eine Auswahleinheit (4) 2x2-Kontingenztafeln auswählt, für welche ein Ergebnis vom exakten Test nach Fisher, welches als signifikant bezeichnet ist, möglicherweise aus der Vielzahl von 2x2-Kontingenztafeln auf der Grundlage eines Parameters ermittelt wird, welcher im Verlauf von Berechnungen zum Bestimmen des Ergebnisses vom exakten Test nach Fisher ermittelt wurde; und
einen Berechnungsschritt (S2), in welchem eine Berechnungseinheit (2) Berechnungen zum Bestimmen des Ergebnisses vom exakten Test nach Fisher für jede der ausgewählten 2x2-Kontingenztafeln durchführt, wobei die Auswahleinheit (4) eine Auswahl der Kontingenztafeln durchführt, während die Frequenzen in der Vielzahl von Kontingenztafeln durch sichere verteilte Berechnung verborgen gehalten werden, wobei a, b, c und d Frequenzen in einer 2x2-Kontingenztafel repräsentieren und T einen Signifikanzlevel repräsentiert, der Parameter, welcher in einer Berechnung im Verlauf des Bestimmens des Ergebnisses vom exakten Test nach Fisher ermittelt wird, $\log(p_a)$ ist, wie durch die untenstehende Formel definiert, und die Auswahleinheit (4) 2x2-Kontingenztafeln mit $\log(p_a) < \log(T)$ oder alternativ mit $\log(p_a) \leq \log(T)$ auswählt,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j - \sum_{j=1}^{c} \log j - \sum_{j=1}^{d} \log j$$

die Vielzahl von Kontingenztafeln eine Vielzahl von 2x2-Kontingenztafeln i (i = 1, 2, ..., m) ist, wobei m eine positive ganze Zahl ist; die Frequenzen in der Kontingenztafel i als $a_i$, $b_i$, $c_i$, $d_i$ dargestellt sind; Informationen, welche durch Verbergen von $a_i$, $b_i$, $c_i$ bzw. $d_i$ unter Verwendung eines verteilten Verschlüsselungsverfahrens erzeugt werden, welches eine sichere verteilte Berechnung eines Größenvergleichs, einer Gleichheitsbestimmung, einer Addition/Subtraktion und einer Multiplikation ermöglicht, während Eingaben und Ausgaben verborgen werden, als $E(a_i)$, $E(b_i)$, $E(c_i)$ bzw. $E(d_i)$ dargestellt werden; eine Information, welche angibt ob die Kontingenztafel i eine 2x2-Kontingenztafel ist, für welche ein Ergebnis vom exakten Test nach Fisher, welches als signifikant bezeichnet ist, möglicherweise ermittelt wird oder nicht, als $X_i'$ dargestellt ist, wobei eine sichere Berechnung eines Größenvergleichs und einer Gleichheitsbestimmung einen Schlüsseltext $E(z)$ für das Ergebnis des Größenvergleichs oder der Gleichheitsbestimmung unter Verwendung von $E(x)$ und y als die Eingabe ohne Entschlüsseln von $E(x)$ bestimmen kann und wobei eine sichere Berechnung der Multiplikation $E(xy)$ aus $E(x)$ und y als die Eingabe ohne Entschlüsseln von $E(x)$ bestimmen kann; und U eine positive ganze Zahl ist,

die Auswahleinheit (4) $E(X_i')$ aus $E(a_i)$, $E(b_i)$, $E(c_i)$, $E(d_i)$ auf der Grundlage von $E(\log(p_a))$ durch sichere Berechnung von Größenvergleichen mit einer Eingabe $E(\log(p_a))$ und $\log(T)$ sicher berechnet, um so m Mengen $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$ zu bestimmen, die m Mengen $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$ sortiert, während $X_i'$ verborgen wird, so dass die Information, welche angibt ob die Kontingenztafel i eine 2x2-Kontingenztafel ist, für welche ein Ergebnis vom exakten Test nach Fisher, welches als signifikant bezeichnet ist, möglicherweise ermittelt wird oder nicht, oben oder am Ende lokalisiert ist, und U Mengen von oben oder vom Ende der m Mengen auswählt, nachdem sie sortiert sind, wobei die Auswahleinheit (4) $E(\log(p_a))$ auf der Grundlage vorberechneter Werte $\sum_{j=1}^{k} \log j$ für k = 0, 1, ..., n berechnet, indem für jeden Summanden in der obenstehenden Formel für $\log(p_a)$ eine sichere Berechnung einer Gleichheitsbestimmung verwendet wird, welche $E(e) = E(1)$, wenn r = k, und andernfalls $E(e) = E(0)$ ergibt, wobei r die Obergrenze des laufenden Indexes j für den jeweiligen Summanden ist, und $E(\sum_{j=1}^{k} \log j)$ für jedes k = 0, 1, ..., n aus $E(e)$ und $\sum_{j=1}^{k} \log j$ durch sichere Berechnung einer Multiplikation berechnet wird, und die ermittelten Ergebnisse für alle k = 0, 1, ..., n addiert werden, während sie verschlüsselt gehalten werden, um einen Schlüsseltext $E(S)$ des jeweiligen Summanden zu ermitteln, wobei S der jeweilige Summand ist, und die Schlüsseltexte der Summanden in der obenstehenden Formel für $\log(p_a)$ addiert werden, während sie verschlüsselt gehalten werden.

6. Computerimplementiertes Verfahren zum Durchführen vom exakten Test nach Fisher für eine Vielzahl von 2x2-Kontingenztafeln, das Verfahren Folgendes umfassend:

einen Auswahlschritt (S4), in welchem eine Auswahleinheit (4) 2x2-Kontingenztafeln auswählt, für welche ein Ergebnis vom exakten Test nach Fisher, welches als signifikant bezeichnet ist, möglicherweise aus der Vielzahl von 2x2-Kontingenztafeln auf der Grundlage eines Parameters ermittelt wird, welcher im Verlauf von Berechnungen zum Bestimmen des Ergebnisses vom exakten Test nach Fisher ermittelt wurde; und
einen Berechnungsschritt (S2), in welchem eine Berechnungseinheit (2) Berechnungen zum Bestimmen des Ergebnisses vom exakten Test nach Fisher für jede der ausgewählten 2x2-Kontingenztafeln durchführt,
wobei die Auswahleinheit (4) eine Auswahl der Kontingenztafeln durchführt, während die Frequenzen in der Vielzahl von Kontingenztafeln durch sichere verteilte Berechnung verborgen gehalten werden,
wobei a, b, c und d Frequenzen in einer 2x2-Kontingenztafel repräsentieren und T einen Signifikanzlevel repräsentiert, der Parameter, welcher in einer Berechnung im Verlauf des Bestimmens des Ergebnisses vom exakten Test nach Fisher ermittelt wird, $\log(p_a)$ ist, wie durch die untenstehende Formel definiert, und die Auswahleinheit (4) 2x2-Kontingenztafeln mit $\log(p_a) < \log(T)$ oder alternativ mit $\log(p_a) \leq \log(T)$ auswählt,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j$$
$$- \sum_{j=1}^{b} \log j - \sum_{j=1}^{c} \log j - \sum_{j=1}^{d} \log j$$

die Vielzahl von Kontingenztafeln eine Vielzahl von 2x2-Kontingenztafeln i (i = 1, 2, ..., m) ist, wobei m eine positive ganze Zahl ist; die Frequenzen in der Kontingenztafel i als $a_i$, $b_i$, $c_i$, $d_i$ dargestellt sind; Informationen,

welche durch Verbergen von $a_i$, $b_i$, $c_i$ bzw. $d_i$ unter Verwendung eines verteilten Verschlüsselungsverfahrens erzeugt werden, welches eine sichere verteilte Berechnung eines Größenvergleichs, einer Gleichheitsbestimmung, einer Addition/Subtraktion und einer Multiplikation ermöglicht, während Eingaben und Ausgaben verborgen werden, als $E(a_i)$, $E(b_i)$, $E(c_i)$ bzw. $E(d_i)$ dargestellt werden; eine Information, welche angibt ob die Kontingenztafel i eine 2x2-Kontingenztafel ist, für welche ein Ergebnis vom exakten Test nach Fisher, welches als signifikant bezeichnet ist, möglicherweise ermittelt wird oder nicht, als $X_i'$ dargestellt ist, wobei eine sichere Berechnung eines Größenvergleichs und eine Gleichheitsbestimmung einen Schlüsseltext $E(z)$ für das Ergebnis des Größenvergleichs oder der Gleichheitsbestimmung unter Verwendung von $E(x)$ und y als die Eingabe ohne Entschlüsseln von $E(x)$ bestimmen kann und wobei eine sichere Berechnung der Multiplikation $E(xy)$ aus $E(x)$ und y als die Eingabe ohne Entschlüsseln von $E(x)$ bestimmen kann,

die Auswahleinheit (4) $E(X_i')$ aus $E(a_i)$, $E(b_i)$, $E(c_i)$, $E(d_i)$ auf der Grundlage von $E(\log(p_a))$ durch sichere Berechnung von Größenvergleichen mit einer Eingabe $E(\log(p_a))$ und $\log(T)$ sicher berechnet, um so m Mengen $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$ zu bestimmen, und, wenn $X_i'$ eine Information, welche darstellt, dass sie keine 2x2-Kontingenztafel ist, für welche ein Ergebnis vom exakten Test nach Fisher, das als signifikant bezeichnet ist, möglicherweise ermittelt wird, $FALSE_i'$ für mindestens eine Menge der m Mengen, $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$ ist, dieses $X_i'$ probabilistisch durch eine Information, welche darstellt, dass sie eine 2x2-Kontingenztafel ist, für welche ein Ergebnis vom exakten Test nach Fisher, das als signifikant bezeichnet ist, möglicherweise ermittelt wird, $TRUE_i'$ ersetzt, während das $X_i'$ verborgen wird, indem m Datenstücke $E(Y_1')$, $E(Y_2')$, ..., $E(Y_m')$, für welche im Voraus TRUE' oder FALSE' probabilistisch verborgen wurde, vorbereitet werden und indem, während $X_i'$ und $Y_i'$ verborgen sind, $Z_i'$ = TRUE', wenn $X_i'$ $TRUE_i'$ repräsentiert oder $Y_i'$ $TRUE_i'$ repräsentiert, und andernfalls $Z_i'$ = $FALSE_i'$ berechnet wird, die Reihenfolge der m Mengen $(E(a_1), E(b_1), E(c_1), E(d_1), E(X_1'))$, $(E(a_2), E(b_2), E(c_2), E(d_2), E(X_2'))$, ..., $(E(a_m), E(b_m), E(c_m), E(d_m), E(X_m'))$ nach dem Ersetzen umordnet, während die umgeordnete Reihenfolge verborgen wird, $E(X_i')$ entschlüsselt und Summentafeln auswählt, für welche ein Ergebnis vom exakten Test nach Fisher, welches angibt, dass ein Ergebnis der Entschlüsselung signifikant ist, möglicherweise ermittelt wird, wobei die Auswahleinheit (4) $E(\log(p_a))$ auf der Grundlage vorberechneter Werte $\sum_{j=1}^{k} \log j$ für k = 0, 1, ..., n berechnet, indem für jeden Summanden in der obenstehenden Formel für $\log(p_a)$ eine sichere Berechnung einer Gleichheitsbestimmung verwendet wird, welche $E(e) = E(1)$, wenn r = k, und andernfalls $E(e) = E(0)$ ergibt, wobei r die Obergrenze des laufenden Indexes j für den jeweiligen Summanden ist, und $E\left(\sum_{j=1}^{k} \log j\right)$ für jedes k = 0, 1, ..., n aus $E(e)$ und $\sum_{j=1}^{k} \log j$ durch sichere Berechnung einer Multiplikation berechnet wird, und die ermittelten Ergebnisse für alle k = 0, 1, ..., n addiert werden, während sie verschlüsselt gehalten werden, um einen Schlüsseltext $E(S)$ des jeweiligen Summanden zu ermitteln, wobei S der jeweilige Summand ist, und die Schlüsseltexte der Summanden in der obenstehenden Formel für $\log(p_a)$ addiert werden, während sie verschlüsselt gehalten werden.

7. Programm zum Veranlassen, dass ein Computer als die Einheiten (2, 4) der Vorrichtung zur Berechnung vom exakten Test nach Fisher nach einem der Ansprüche 1 bis 3 funktioniert.

**Revendications**

1. Appareil de calcul de test exact de Fisher pour effectuer le test exact de Fisher pour une pluralité de tableaux de contingence 2x2, l'appareil comprenant :

   une unité de sélection (4) qui est adaptée pour sélectionner des tableaux de contingence 2x2 pour lesquels un résultat du test exact de Fisher indiquant qu'il est significatif sera éventuellement obtenu parmi la pluralité de tableaux de contingence 2x2 sur la base d'un paramètre obtenu au cours de calculs pour déterminer le résultat du test exact de Fisher ; et
   une unité de calcul (2) qui est adaptée pour effectuer des calculs pour déterminer le résultat du test exact de Fisher pour chacun des tableaux de contingence 2x2 sélectionnés,
   dans lequel l'unité de sélection (4) est adaptée pour effectuer la sélection des tableaux de contingence tout en gardant les fréquences dans la pluralité de tableaux de contingence masquées via un calcul sécurisé multipartite, où a, b, c et d représentent les fréquences dans un tableau de contingence 2x2 et T représente le niveau de signification, le paramètre obtenu dans le calcul au cours de la détermination du résultat du test exact de Fisher est $\log(p_a)$ défini par la formule ci-dessous, et

l'unité de sélection (4) est adaptée pour sélectionner des tableaux de contingence 2x2 avec $\log(p_a)<\log(T)$ ou, alternativement, $\log(p_a) \leq \log(T)$,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j - \sum_{j=1}^{c} \log j - \sum_{j=1}^{d} \log j$$

la pluralité de tableaux de contingence est une pluralité de tableaux de contingence 2x2 i(i=1, 2, ..., m), où m est un entier positif ; les fréquences dans le tableau de contingence i sont représentés par $a_i$, $b_i$, $c_i$, $d_i$ ; les informations générées en masquant $a_i$, $b_i$, $c_i$, $d_i$, respectivement, en utilisant une technique de cryptage multipartite permettant un calcul sécurisé multipartite de comparaison de grandeur, de détermination d'égalité, d'addition/soustraction et de multiplication, tout en masquant l'entrée et la sortie, est représenté par $E(a_i)$, $E(b_i)$, $E(c_i)$, $E(d_i)$ ; et des informations indiquant si le tableau de contingence i est un tableau de contingence 2x2 pour lequel un résultat du test exact de Fisher indiquant qu'il est significatif sera éventuellement obtenu ou non est représenté par $X_i'$, dans lequel le calcul sécurisé de comparaison de grandeur et de détermination d'égalité peut déterminer le texte crypté E(z) pour le résultat de comparaison de grandeur ou de détermination d'égalité en utilisant E(x) et y comme entrée sans décrypter E(x), et dans lequel un calcul sécurisé de multiplication peut déterminer E(xy) à partir de E(x) et y comme entrée sans décrypter E(x),

l'unité de sélection (4) est adaptée pour calculer en toute sécurité $E(X_i')$ à partir de $E(a_i)$, $E(b_i)$, $E(c_i)$, $E(d_i)$ basé sur $E(\log(p_a))$ par calcul sécurisé de comparaison de grandeur avec les entrées $E(\log(p_a))$ et $\log(T)$ pour déterminer m ensembles, (E(ai), E(bi), E(ci), E(Xi')), (E(a2), E(b2), E(c2), E(d2), E (X2')),..., (E(am), E(bm), E (Cm) , E (dm), E(Xm')), et mélanger un ordre des m ensembles, (E(a1), E(b1), E(c1)), (E(a2), E(b2), E(c2), E(d2), E(X2')), (E(am), E(bm), E(cm), E(dm), E(Xm')), tout en masquant l'ordre mélangé, décrypter $E(X_i')$, et sélectionner des tableaux de contingence 2x2 pour lesquels un résultat de test exact de Fisher indiquant qu'un résultat du décryptage est significatif, sera éventuellement obtenu, dans lequel l'unité de sélection (4) est adaptée pour calculer $E(\log(p_a))$ sur la base des valeurs précalculées $\sum_{j=1}^{k} \log j$ pour k=0, 1, n, en, pour chaque somme dans la formule de $\log(p_a)$ ci-dessus, utilisant un calcul sécurisé pour déterminer l'égalité qui retourne E(e)=E(1) si r=k et E(e)=E(0) sinon, où r est la limite supérieure de l'indice courant j pour la somme respective, et calculer $E(\sum_{j=1}^{k} \log j)$ pour chaque k=0, 1,...,n de E(e) et $E(\sum_{j=1}^{k} \log j)$ par calcul sécurisé de multiplication, et additionner les résultats obtenus pour chaque k=0, 1,...,n, tout en les gardant cryptés pour obtenir le texte crypté E(S) de la somme respective, où S est la somme respective, et en additionnant les textes cryptés des sommes dans la formule pour $\log(P_a)$ ci-dessus tout en les gardant cryptés.

2. Appareil de calcul de test exact de Fisher pour effectuer le test exact de Fisher pour une pluralité de tableaux de contingence 2x2, l'appareil comprenant :

une unité de sélection (4) qui est adaptée pour sélectionner des tableaux de contingence 2x2 pour lesquels un résultat du test exact de Fisher indiquant qu'il est significatif sera éventuellement obtenu parmi la pluralité de tableaux de contingence 2x2 sur la base d'un paramètre obtenu au cours de calculs pour déterminer le résultat du test exact de Fisher ; et

une unité de calcul qui est adaptée pour effectuer des calculs pour déterminer le résultat du test exact de Fisher pour chacun des tableaux de contingence 2x2 sélectionnés, où l'unité de sélection (4) est adaptée pour effectuer la sélection des tableaux de contingence tout en gardant les fréquences dans la pluralité de tableaux de contingence masquées via un calcul sécurisé multipartite,

où a, b, c et d représentent les fréquences dans un tableau de contingence 2x2 et T représente le niveau de signification, le paramètre obtenu lors du calcul au cours de la détermination du résultat du test exact de Fisher est $\log(p_a)$ défini par la formule ci-dessous, et

l'unité de sélection (4) est adaptée pour sélectionner des tableaux de contingence 2x2 avec log($p_a$)<log(T) ou, alternativement, log($p_a$) $\leq$ log(T),

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j - \sum_{j=1}^{c} \log j - \sum_{j=1}^{d} \log j$$

la pluralité de tableaux de contingence est une pluralité de tableaux de contingence 2x2 i(i=1, 2, ..., m), où m est un entier positif ; les fréquences dans le tableau de contingence i sont représentés par $a_i$, $b_i$, $c_i$, $d_i$ ; les informations générées en masquant $a_i$, $b_i$, $c_i$, $d_i$, respectivement, en utilisant une technique de cryptage multipartite permettant un calcul sécurisé multipartite de comparaison de grandeur, de détermination d'égalité, d'addition/soustraction et de multiplication, tout en masquant l'entrée et la sortie, est représenté par $E(a_i)$, $E(b_i)$, $E(c_i)$, $E(d_i)$; et des informations indiquant si le tableau de contingence i est un tableau de contingence 2x2 pour lequel un résultat du test exact de Fisher indiquant qu'il est significatif sera éventuellement obtenu ou non est représenté par $X_i'$, dans lequel le calcul sécurisé de comparaison de grandeur et de détermination d'égalité peut déterminer le texte crypté E(z) pour le résultat de comparaison de grandeur ou de détermination d'égalité en utilisant E (x) et y comme entrée sans décrypter E(x), et dans lequel un calcul sécurisé de multiplication peut déterminer E(xy) à partir de E(x) et y comme entrée sans décrypter E(x) ; et U est un entier positif, l'unité de sélection (4) est adaptée pour calculer en toute sécurité $E(X_i')$ à partir de $E(a_i)$, $E(b_i)$, $E(c_i)$, $E(d_i)$ basé sur $E(\log(p_a))$ par calcul sécurisé de comparaison de grandeur avec les entrées $E(\log(p_a))$ et log(T) pour déterminer m ensembles, (E(ai), E(bi), E(ci), E(Xi')), (E(a2), E(b2), E(c2), E(d2), E(X2') ),..., (E(am), E($b_m$), E(Cm) , E(dm), E(Xm')), trier les m ensembles, (E(a1), E(b1), E(c1)), (E(a2), E(b2), E(c2), E(d2), E(X2')), (E(am), E(bm), E(cm), E(dm), E(Xm')), tout en masquant $X_i'$, tout en masquant $X_i'$ de sorte que l'information indiquant si le tableau de contingence i est un tableau de contingence 2x2 pour lequel un résultat du test exact de Fisher indiquant qu'il est significatif sera éventuellement obtenu ou non est situé à un sommet ou à une fin, et sélectionner les ensembles U au sommet ou à la fin des m ensembles après qu'ils aient été triés, dans lequel l'unité de sélection (4) est adaptée pour calculer $E(\log(p_a))$ sur la base des valeurs précalculées $\sum_{j=1}^{k} \log j$ pour k=0, 1, n, en, pour chaque somme dans la formule de log($p_a$) ci-dessus, utilisant un calcul sécurisé pour déterminer l'égalité qui retourne E(e)=E(1) si r=k et E(e)=E(0) sinon, où r est la limite supérieure de l'indice courant j pour la somme respective, et calculer $E(\sum_{j=1}^{k} \log j)$ pour chaque k=0, 1, n de E(e) et $\sum_{j=1}^{k} \log j$ par calcul sécurisé de multiplication, et additionner les résultats obtenus pour chaque k=0, 1,...,n, tout en les gardant cryptés pour obtenir le texte crypté E(S) de la somme respective, où S est la somme respective, et en additionnant les textes cryptés des sommes dans la formule pour log($P_a$) ci-dessus tout en les gardant cryptés.

3. Appareil de calcul de test exact de Fisher pour effectuer le test exact de Fisher pour une pluralité de tableaux de contingence 2x2, l'appareil comprenant :

une unité de sélection (4) qui est adaptée pour sélectionner des tableaux de contingence 2x2 pour lesquels un résultat du test exact de Fisher indiquant qu'il est significatif sera peut-être obtenu parmi la pluralité de tableaux de contingence 2x2 sur la base d'un paramètre obtenu au cours des calculs pour déterminer le résultat du test exact de Fisher ; et

une unité de calcul (4) qui est adaptée pour effectuer des calculs pour déterminer le résultat du test exact de Fisher pour chacun des tableaux de contingence 2x2 sélectionnés,

dans lequel l'unité de sélection (4) est adaptée pour effectuer la sélection des tableaux de contingence tout en gardant les fréquences dans la pluralité de tableaux de contingence masquées via un calcul sécurisé multipartite, où a, b, c et d représentent les fréquences dans un tableau de contingence 2x2 et T représente le niveau de signification, le paramètre obtenu lors du calcul au cours de la détermination du résultat du test exact de Fisher est log($p_a$) défini par la formule ci-dessous, et

l'unité de sélection (4) est adaptée pour sélectionner des tableaux de contingence 2x2 avec log($p_a$)<log(T) ou, alternativement, log ($p_a$) $\leq$ log (T),

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j$$
$$- \sum_{j=1}^{c} \log j - \sum_{j=1}^{d} \log j$$

la pluralité de tableaux de contingence est une pluralité de tableaux de contingence 2x2 i (1 = 1, 2,..., m) , où m est un entier positif ; les fréquences dans le tableau de contingence i sont représentées par $a_i$, $b_i$, $c_i$, $d_i$ ; les informations générées en masquant $a_i$, $b_i$, $d_i$, respectivement, en utilisant une technique de cryptage multipartite permettant le calcul sécurisé de comparaison de grandeur, de détermination d'égalité, d'addition/soustraction et de multiplication, tout en masquant l'entrée et la sortie, sont représentées par $E(a_i)$, $E(b_i)$, $E(c_i)$, $E(d_1)$, respectivement ; et des informations indiquant si le tableau de contingence i est un tableau de contingence 2x2 pour lequel un résultat du test exact de Fisher indiquant qu'il est significatif sera éventuellement obtenu ou non est représenté comme $X_i$', dans lequel le calcul sécurisé de comparaison de grandeur et de détermination d'égalité peut déterminer le texte crypté $E(z)$ pour le résultat de comparaison de grandeur ou de détermination d'égalité en utilisant $E(x)$ et y comme entrée sans décrypter $E(x)$, et dans lequel un calcul sécurisé de multiplication peut déterminer $E(xy)$ à partir de $E(x)$ et y comme entrée sans décrypter $E(x)$,

l'unité de sélection (4) est adaptée pour calculer en toute sécurité $E(X_i')$ à partir de $E(a_i)$, $E(b_i)$, $E(c_i)$, $E(d_i)$ basé sur $E(\log(p_a))$ par calcul sécurisé de comparaison de grandeur avec l'entrée $E(\log(p_a))$ et $\log(T)$ pour déterminer m ensembles, $(E(ai), E(bi), E(ci), E(d1), E(X1'))$, $(E(a2), E(b2), E(c2), E(d2), E(X2'))$, $(E(am), E(bm), E(Cm), E(dm), E(Xm'))$, et si $X_i$' est une information qui indique qu'il ne s'agit pas d'un tableau de contingence 2x2 pour lequel un résultat du test exact de Fisher indiquant qu'il est significatif sera peut-être obtenu, FAUX$i$' ; pour au moins un ensemble parmi les m ensembles, $(E(a1), E(b1), E(c1), E(d1), E(X1'))$, $(E(a2), E(b2), E(c2), E(d2), E(X2'))$, $(E(am), E(bm), E(Cm), E(dm), E(Xm'))$, remplacer de manière probabiliste ce $X_i$' par des informations représentant un tableau de contingence 2x2 pour lequel un résultat du test exact de Fisher indiquant qu'il est significatif sera éventuellement obtenu, VRAI, tout en masquant le $X_i$', en préparant m données $E(Y1')$, $E(Y2')$, $E(Ym')$ pour lesquelles VRAI' ou FAUX' est masqué de manière probabiliste à l'avance, et en, tout en masquant Xi' et Yi', calculant Z,'=VRAI' si Xi' représente VRAI ou Yi' représente VRAI, et Zi'=FAUX sinon, mélanger l'ordre des m ensembles, $(E(a1), E(b1), E(c1), E(X1'))$, $(E(a2), E(b2), E(c2), E(d2), E(X2'))$, $(E(am), E(bm), E(cm), E(dm), E(Xm'))$, après le remplacement tout en masquant l'ordre mélangé, décrypter $E(X_i')$, et sélectionner des tableaux récapitulatifs pour lesquels un résultat du test exact de Fisher indiquant qu'un résultat du décryptage est significatif sera éventuellement obtenu, dans lequel l'unité de sélection (4) est adaptée pour

calculer $E(\log(p_a))$ à partir des valeurs précalculées $\sum_{j=1}^{k} \log j \mathrm{E}$ pour k=0, 1,...,n, en, pour chaque somme dans la formule pour $\log(p_a)$ ci-dessus, utilisant un calcul sécurisé de détermination d'égalité qui retourne $E(e)=E(1)$ si r=k et $E(e)=E(0)$ sinon, où r est la limite supérieure de l'indice courant j pour la somme, et calculer

$E(\sum_{j=1}^{k} \log j)$ pour chaque k=0, 1, n à partir de $E(e)$ et $\sum_{j=1}^{k} \log j$ par calcul sécurisé de la multiplication, et additionner les résultats pour tout k=0, 1,...,n tout en les gardant cryptés pour obtenir un texte crypté $E(S)$ de la somme respective, où S est la somme respective, et en additionnant les textes cryptés des sommes dans la formule pour $\log(p_a)$ ci-dessus en les gardant cryptés.

4. Procédé informatisé d'exécution du test exact de Fisher pour une pluralité de tableaux de contingence 2x2, le procédé comprenant :

une étape de sélection (S4) dans laquelle une unité de sélection (4) sélectionne des tableaux de contingence 2x2 pour lesquels un résultat du test exact de Fisher indiquant qu'il est significatif sera éventuellement obtenu parmi la pluralité de tableaux de contingence 2x2 sur la base d'un paramètre obtenu au cours de calculs pour déterminer le résultat du test exact de Fisher ; et
une étape de calcul (S2) dans laquelle une unité de calcul(2) effectue des calculs pour déterminer le résultat du test exact de Fisher pour chacun des tableaux de contingence 2x2 sélectionnés,
dans lequel l'unité de sélection (4) effectue la sélection des tableaux de contingence tout en gardant les fréquences dans la pluralité de tableaux de contingence masquées via un calcul sécurisé multipartite,
où a, b, c et d représentent les fréquences dans un tableau de contingence 2x2 et T représente le niveau de signification, le paramètre obtenu lors du calcul au cours de la détermination du résultat du test exact de Fisher est $\log(p_a)$ défini par la formule ci-dessous, et

l'unité de sélection (4) sélectionne des tableaux de contingence 2x2 avec $\log(p_a)<\log(T)$ ou, alternativement, $\log(p_a)\leq\log(T)$,

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j$$
$$- \sum_{j=1}^{c} \log j - \sum_{j=1}^{d} \log j$$

la pluralité de tableaux de contingence est une pluralité de tableaux de contingence 2x2 i (i=1, 2, ..., m), où m est un entier positif ; les fréquences dans le tableau de contingence i sont représentées par $a_i$, $b_i$, $c_i$, $d_i$ ; les informations générées en masquant $a_i$, $b_i$, $c_i$, $d_i$, respectivement, en utilisant une technique de cryptage multipartite permettant le calcul sécurisé de comparaison de grandeur, de détermination d'égalité, d'addition/soustraction et de multiplication, tout en masquant l'entrée et la sortie, est représenté par $E(a_i)$, $E(b_i)$, $E(c_i)$, $E(d_i)$, respectivement ; et des informations indiquant si le tableau de contingence i est un tableau de contingence 2x2 pour lequel un résultat du test exact de Fisher indiquant qu'il est significatif sera éventuellement obtenu ou non, sont représentées par $X_i'$, dans lequel un calcul sécurisé de comparaison de grandeur et de détermination d'égalité peut déterminer un texte crypté $E(z)$ pour le résultat de comparaison de grandeur ou de détermination d'égalité en utilisant $E(x)$ et y comme entrée sans décrypter $E(x)$, et dans lequel le calcul sécurisé de la multiplication peut déterminer $E(xy)$ à partir de $E(x)$ et y comme entrée sans décrypter $E(x)$,

l'unité de sélection (4) calcule en toute sécurité $E(X_i')$ à partir de $E(ai)$, $E(bi)$, $E(ci)$, $E(di)$ sur $E(\log(p_a))$ par calcul sécurisé des comparaisons de grandeur avec l'entrée $E(\log(p_a))$ et $\log(T)$ pour déterminer m ensembles, $(E(a1), E(b1), E(c1),E(X1'))$, $(E(a2), E(b2), E(c2), E(d2), E(X2'))$,..., $(E(am), E(bm), E(Cm), E(dm), E(Xm'))$, et mélanger un ordre des m ensembles, $(E(a1), E(b1), E(c1))$, $(E(a2), E(b2), E(c2), E(d2), E(X2'))$, $(E(am), E(bm), E(cm), E(dm), E(Xm'))$, tout en masquant l'ordre mélangé, décrypter $E(X_i')$, et sélectionner des tableaux de contingence 2x2 pour lesquels un résultat de test exact de Fisher indiquant qu'un résultat du décryptage est significatif, sera éventuellement obtenu, dans lequel l'unité de sélection (4) est adaptée pour calculer $E(\log(p_a))$ sur la base des valeurs précalculées $\sum_{j=1}^{k} \log j$ pour k=0, 1, n, en, pour chaque somme dans la formule de $\log(p_a)$ ci-dessus, utilisant un calcul sécurisé pour déterminer l'égalité qui retourne $E(e)=E(1)$ si r=k et $E(e)=E(0)$ sinon, où r est la limite supérieure de l'indice courant j pour la somme respective, et calculer $E(\sum_{j=1}^{k} \log j)$ pour chaque k=0, 1,...,n de $E(e)$ et $\sum_{j=1}^{k} \log j$ par calcul sécurisé de multiplication, et additionner les résultats obtenus pour chaque k=0, 1,...,n, tout en les gardant cryptés pour obtenir le texte crypté $E(S)$ de la somme respective, où S est la somme respective, et en additionnant les textes cryptés des sommes dans la formule pour $\log(P_a)$ ci-dessus tout en les gardant cryptés.

5. Procédé mis en œuvre par ordinateur pour effectuer le test exact de Fisher pour une pluralité de tableaux de contingence 2x2, le procédé comprenant :

une étape de sélection (S4) dans laquelle une unité de sélection (4) sélectionne des tableaux de contingence 2x2 pour lesquels un résultat du test exact de Fisher indiquant qu'il est significatif sera éventuellement obtenu parmi la pluralité de tableaux de contingence 2x2 sur la base d'un paramètre obtenu au cours de calculs pour déterminer le résultat du test exact de Fisher ; et

une étape de calcul (S2) dans laquelle une unité de calcul (2) effectue des calculs pour déterminer le résultat du test exact de Fisher pour chacun des tableaux de contingence 2x2 sélectionnés,

dans lequel l'unité de sélection (4) effectue la sélection des tableaux de contingence tout en gardant les fréquences dans la pluralité de tableaux de contingence masquées via un calcul sécurisé multipartite,

où a, b, c et d représentent les fréquences dans un tableau de contingence 2x2 et T représente le niveau de signification, le paramètre obtenu dans le calcul au cours de la détermination du résultat du test exact de Fisher est $\log(p_a)$ défini par la formule ci-dessous, et

l'unité de sélection (4) sélectionne des tableaux de contingence 2x2 avec $\log(p_a)<\log(T)$ ou, alternativement,

log(p$_a$) ≤log(T),

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j$$
$$- \sum_{j=1}^{c} \log j - \sum_{j=1}^{d} \log j$$

la pluralité de tableaux de contingence est une pluralité de tableaux de contingence 2x2 i(i=1, 2, ..., m), où m est un entier positif ; les fréquences dans le tableau de contingence i sont représentés par $a_i$, $b_i$, $c_i$, $d_i$ ; les informations générées en masquant $a_i$, $b_i$, $c_i$, $d_i$, respectivement, en utilisant une technique de cryptage multipartite permettant un calcul sécurisé multipartite de comparaison de grandeur, de détermination d'égalité, d'addition/soustraction et de multiplication, tout en masquant l'entrée et la sortie, est représenté par $E(a_i)$, $E(b_i)$, $E(c_i)$, $E(d_i)$ ; et des informations indiquant si le tableau de contingence i est un tableau de contingence 2x2 pour lequel un résultat du test exact de Fisher indiquant qu'il est significatif sera éventuellement obtenu ou non est représenté par $X_i'$, dans lequel le calcul sécurisé de comparaison de grandeur et de détermination d'égalité peut déterminer le texte crypté E(z) pour le résultat de comparaison de grandeur ou de détermination d'égalité en utilisant E(x) et y comme entrée sans décrypter E(x), et dans lequel un calcul sécurisé de multiplication peut déterminer E(xy) à partir de E(x) et y comme entrée sans décrypter E(x) ; et U est un entier positif, l'unité de sélection (4) est adaptée pour calculer en toute sécurité $E(X_i')$ à partir de $E(a_i)$, $E(b_i)$, $E(c_i)$, $E(d_i)$ basé sur $E(\log(p_a))$ par calcul sécurisé de comparaison de grandeur avec les entrées $E(\log(p_a))$ et log(T) pour déterminer m ensembles, (E(ai), E(bi), E(ci), E(Xi')), (E(a2), E(b2), E(c2), E(d2), E(X2')),..., (E(am), E(bm), E (Cm), E(dm), E(Xm')), trier les m ensembles, (E(a1), E(b1), E(c1)), (E(a2), E(b2), E(c2), E(d2), E(X2')), (E(am), E(bm), E(cm), E(dm), E(Xm')), tout en masquant $X_i'$, tout en masquant $X_i'$ de sorte que l'information indiquant si le tableau de contingence i est un tableau de contingence 2x2 pour lequel un résultat du test exact de Fisher indiquant qu'il est significatif sera éventuellement obtenu ou non est situé à un sommet ou à une fin, et sélectionner les ensembles U au sommet ou à la fin des m ensembles après qu'ils aient été triés, dans lequel l'unité de sélection (4) est adaptée pour calculer E (log ($p_a$)) sur la base des valeurs précalculées $\sum_{j=1}^{k} \log j$ pour k=0, 1, n, en, pour chaque somme dans la formule de log($p_a$) ci-dessus, utilisant un calcul sécurisé pour déterminer l'égalité qui retourne E(e)=E(1) si r=k et E(e)=E(0) sinon, où r est la limite supérieure de l'indice courant j pour la somme respective, et calculer $E(\sum_{j=1}^{k} \log j)$ pour chaque k=0, 1, n de E(e) et $\sum_{j=1}^{k} \log j$ par calcul sécurisé de multiplication, et additionner les résultats obtenus pour chaque k=0, 1,...,n, tout en les gardant cryptés pour obtenir le texte crypté E(S) de la somme respective, où S est la somme respective, et en additionnant les textes cryptés des sommes dans la formule pour log($P_a$) ci-dessus tout en les gardant cryptés.

6. Procédé informatisé d'exécution du test exact de Fisher pour une pluralité de tableaux de contingence 2x2, le procédé comprenant :

une étape de sélection (S4) dans laquelle une unité de sélection (4) sélectionne 2x2 tableaux de contingence pour lesquels un résultat du test exact de Fisher indiquant qu'il est significatif sera éventuellement obtenu parmi la pluralité de tableaux de contingence 2x2 basés sur un paramètre obtenu au cours de calculs pour déterminer le résultat du test exact de Fisher ; et
une étape de calcul (S2) dans laquelle une unité de calcul (2) effectue des calculs pour déterminer le résultat du test exact de Fisher pour chacun des tableaux de contingence 2x2 sélectionnés,
dans lequel l'unité de sélection (4) effectue la sélection des tableaux de contingence tout en gardant les fréquences dans la pluralité de tableaux de contingence masquées via un calcul sécurisé multipartite,
où a, b, c et d représentent les fréquences dans un tableau de contingence 2x2 et T représente le niveau de signification, le paramètre obtenu lors du calcul au cours de la détermination du résultat du test exact de Fisher est log($p_a$) défini par la formule ci-dessous, et
l'unité de sélection (4) sélectionne des tableaux de contingence 2x2 avec log($p_a$)<log(T) ou, alternativement, log(p$_a$) ≤log(T),

$$\log p_a = \sum_{j=1}^{a+b} \log j + \sum_{j=1}^{c+d} \log j + \sum_{j=1}^{a+c} \log j + \sum_{j=1}^{b+d} \log j - \sum_{j=1}^{n} \log j - \sum_{j=1}^{a} \log j - \sum_{j=1}^{b} \log j$$
$$- \sum_{j=1}^{c} \log j - \sum_{j=1}^{d} \log j$$

la pluralité de tableaux de contingence est une pluralité de tableaux de contingence 2x2 i (i = 1, 2,..., m) , où m est un entier positif ; les fréquences dans le tableau de contingence i sont représentées par $a_i$, $b_i$, $c_i$, $d_i$ ; les informations générées en masquant $a_i$, $b_i$, $d_i$, respectivement, en utilisant une technique de cryptage multipartite permettant le calcul sécurisé de comparaison de grandeur, de détermination d'égalité, d'addition/soustraction et de multiplication, tout en masquant l'entrée et la sortie, sont représentées par $E(a_i)$, $E(b_i)$, $E(c_i)$, $E(d_1)$, respectivement ; et des informations indiquant si le tableau de contingence i est un tableau de contingence 2x2 pour lequel un résultat du test exact de Fisher indiquant qu'il est significatif sera éventuellement obtenu ou non est représenté comme $X_i'$, dans lequel le calcul sécurisé de comparaison de grandeur et de détermination d'égalité peut déterminer le texte crypté $E(z)$ pour le résultat de comparaison de grandeur ou de détermination d'égalité en utilisant $E(x)$ et y comme entrée sans décrypter $E(x)$, et dans lequel un calcul sécurisé de multiplication peut déterminer $E(xy)$ à partir de $E(x)$ et y comme entrée sans décrypter $E(x)$, l'unité de sélection (4) est adaptée pour calculer de manière sécurisée $E(X_i')$ à partir de $E(ai)$, $E(bi)$, $E(ci)$, $E(di)$ basé sur $E(\log(p_a))$ par calcul sécurisé de comparaison de grandeur avec l'entrée $E(\log(p_a))$ et $\log(T)$ pour déterminer m ensembles, $(E(ai), E(bi), E(ci), E(d1), E(X1'))$, $(E(a2), E(b2), E(c2), E(d2), E(X2'))$, $(E(am), E(bm), E(Cm), E(dm), E(Xm'))$, et si $X_i'$ est une information qui indique qu'il ne s'agit pas d'un tableau de contingence 2x2 pour lequel un résultat du test exact de Fisher indiquant qu'il est significatif sera peut-être obtenu, FAUX$i'$ ; pour au moins un ensemble parmi les m ensembles, $(E(a1), E(b1), E(c1), E(d1), E(X1'))$, $(E(a2), E(b2), E(c2), E(d2), E(X2'))$, $(E(am), E(bm), E(Cm), E(dm), E(Xm'))$, remplacer de manière probabiliste ce $X_i'$ par des informations représentant un tableau de contingence 2x2 pour lequel un résultat du test exact de Fisher indiquant qu'il est significatif sera éventuellement obtenu, VRAI, tout en masquant le $X_i'$, en préparant m données $E(Y1')$, $E(Y2')$, $E(Ym')$ pour lesquelles VRAI' ou FAUX' est masqué de manière probabiliste à l'avance, et en, tout en masquant $Xi'$ et $Yi'$, calculant $Z_i'=VRAI'$ si $X_i'$ représente VRAI ou $Yi'$ représente VRAI, et $Zi'=FAUX$ sinon, mélanger l'ordre des m ensembles, $(E(a1), E(b1), E(c1), E(X1'))$, $(E(a2), E(b2), E(c2), E(d2), E(X2'))$, $(E(am), E(bm), E(cm), E(dm), E(Xm'))$, après le remplacement tout en masquant l'ordre mélangé, décrypter $E(X_i')$, et sélectionner des tableaux récapitulatifs pour lesquels un résultat du test exact de Fisher indiquant qu'un résultat du déchryptage est significatif sera éventuellement obtenu, dans lequel l'unité de sélection (4) est adaptée pour

calculer $E(\log(p_a))$ à partir des valeurs précalculées $\sum_{j=1}^{k} \log j$ pour k=0, 1,...,n, en, pour chaque somme dans la formule pour $\log(pa)$ ci-dessus, utilisant un calcul sécurisé de détermination d'égalité qui retourne $E(e)=E(1)$ si r=k et $E(e)=E(0)$ sinon, où r est la limite supérieure de l'indice courant j pour la somme, et calculer $E(\sum_{j=1}^{k} \log j)$ pour chaque k=0, 1, n à partir de $E(e)$ et $\sum_{j=1}^{k} \log j$ par calcul sécurisé de la multiplication, et additionner les résultats pour tout k=0, 1,...,n tout en les gardant cryptés pour obtenir un texte crypté $E(S)$ de la somme respective, où S est la somme respective, et en additionnant les textes cryptés des sommes dans la formule pour $\log(p_a)$ ci-dessus en les gardant cryptés.

**7.** Programme pour amener un ordinateur à fonctionner comme les unités (2,4) de l'appareil de calcul de test exact selon une quelconque des revendications 1 à 3.

SELECTION
UNIT 4

CALCULATION
UNIT 2

FIG. 1

START

SELECT SUMMARY TABLES FOR
WHICH RESULT OF FISHER'S EXACT
TEST INDICATIVE OF BEING SIGNIFICANT
WILL BE POSSIBLY OBTAINED

S4

CALCULATE FISHER'S EXACT TEST FOR
SELECTED SUMMARY TABLE

S2

END

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KONRAD KARCZEWSKI.** How to do a GWAS. *GENE 210: Genomics and Personalized Medicine,* 2015
- **YIHUA ZHANG ; MARINA BLANTON ; GHADA AL-MASHAQBEH.** Secure distributed genome analysis for GWAS and sequence comparison computation. *BMC medical informatics and decision making,* 2015, vol. 15 (5), S4
- **DAN BOGDANOV ; LIINA KAMM ; SVEN LAUR ; VILLE SOKK.** Rmind: a tool for cryptographically secure statistical analysis. *IACR, INTERNATIONAL ASSOCIATION FOR CRYPTOLOGIC RESEARCH,* 30 June 2014, 1-35

- Unconditionally secure constant-rounds multi-party computation for equality, comparison, bits and exponentiation. **IVAN DAMGARD ; MATTHIAS FITZI ; EIKE KILTZ ; JESPER BUUS NIELSEN ; TOMAS TOFT.** Proc. 3rd Theory of Cryptography Conference, TCC 2006. Springer-Verlag, 2006, vol. 3876, 285-304
- **TAKASHI NISHIDE ; KAZUO OHTA.** Multiparty Computation for Interval, Equality, and Comparison Without Bit-Decomposition Protocol. *Public Key Cryptography - PKC 2007, 10th International Conference on Practice and Theory in Public-Key Cryptography,* 2007, 343-360